# EUROPEAN PATENT APPLICATION

(11) **EP 4 059 495 A1**
(43) Date of publication of application: **21.09.2022**
(21) Application number: 21163448.0
(22) Date of filing: 18.03.2021
(51) Int. Cl.: A61K 31/185, A61P 35/00

(54) **THERAPEUTIC COMBINATIONS INCLUDING INHIBITORS OF THE P2X4 RECEPTOR FOR TREATING AND PREVENTING PROLIFERATIVE DISORDERS**

(71) Applicant: Chemotherapeutisches Forschungsinstitut Georg-Speyer-Haus, 60596 Frankfurt am Main (DE)
(72) Inventor: Greten, Florian, 61352 Bad Homburg (DE); Schmitt, Mark, 55131 Mainz (DE); Gupta, Jalaj, 60596 Frankfurt am Main (DE)
(74) Representative: Krauss, Jan

(57) **Abstract**

The present invention relates to the use of inhibitors of the P2X4 receptor or inhibitors of the P2X4 receptor signaling pathway in combination with an effective amount of a "cell death inducing chemotherapeutic drug" or "cell death inducing therapy" in the prevention and/or treatment of a solid tumor or metastases thereof in a subject, in particular in colorectal cancer (CRC).

## Description

The present invention relates to the use of inhibitors of the P2X4 receptor or inhibitors of the P2X4 receptor signaling pathway in combination with an effective amount of a cell death inducing chemotherapeutic drug and/or a cell death inducing therapy in the prevention and/or treatment of a solid tumor or metastases thereof in a subject, in particular in colorectal cancer (CRC).

### Background of the invention

Leucine-rich repeat-containing G-protein-coupled receptor 5 (LGR5) is an intestinal stem cell marker. LGR5 is also a candidate marker for colorectal cancer (CRC) stem cells and may be closely involved in the progression and prognosis of CRC, one of the most common carcinomas worldwide (Wu XS, Xi HQ, Chen L. Lgr5 is a potential marker of colorectal carcinoma stem cells that correlates with patient survival. World J Surg Oncol. 2012;10:244).

Lgr5+ cells have been described as cell of origin and cancer stem cells in colorectal cancer (CRC). Silencing of LGR5 reduced proliferation, migration and colony formation in vitro and tumorigenicity in vivo [Hirsch D, Barker N, McNeil N, Hu Y, Camps J, McKinnon K, Clevers H, Ried T, Gaiser T. LGR5 positivity defines stem-like cells in colorectal cancer. Carcinogenesis. 2014 Apr;35(4):849-58. doi: 10.1093/carcin/bgt377. Epub 2013 Nov 26]. Unfortunately, temporary deletion of Lgr5+ cells in subcutaneous CRC tumors only led to tumor stasis during the days of absence of Lgr5+ cells, followed by a rapid continuation of tumor growth once the Lgr5+ cell pool was re-established (3).

Morgan, R., et al. (in: Targeting LGR5 in Colorectal Cancer: therapeutic gold or too plastic?. Br J Cancer 118, 1410-1418 (2018). https://doi.org/10.1038/s41416-018-0118-6) describe that the well characterised deregulation of Wnt/β-catenin signalling that occurs during the adenoma-carcinoma sequence in colorectal cancer (CRC) renders LGR5 an interesting therapeutic target. Furthermore, recent studies demonstrating that CRC tumours contain LGR5+ subsets and retain a degree of normal tissue architecture has heightened translational interest. Such reports fuel hope that specific subpopulations or molecules within a tumour may be therapeutically targeted to prevent relapse and induce long-term remissions.

Hsu HC, et al. (in: Overexpression of Lgr5 correlates with resistance to 5-FU-based chemotherapy in colorectal cancer. Int J Colorectal Dis. 2013 Nov;28(11):1535-46. doi: 10.1007/s00384-013-1721-x. Epub 2013 Jun 20) disclose that elevated Lgr5 level is associated with CRC progression and treatment response and has the potential to serve as a therapeutic target in CRC patients.

The mammalian target of rapamycin (mTOR) is a serine-threonine kinase that regulates cell growth and proliferation in response to the availability of growth factors and nutrients. Treatment with rapamycin significantly decreased proliferation of certain CRC cell lines (rapamycin sensitive), whereas other cell lines were resistant to its effects (rapamycin resistant). Studies demonstrate limited clinical activity of everolimus for the treatment of advanced colorectal cancer and have been complicated by increases in toxicity. Because of the central role of the PI3K/mTOR pathway in cancer biology other drug combinations with mTOR inhibition have been proposed (Altomare I, Hurwitz H. Everolimus in colorectal cancer. Expert Opin Pharmacother. 2013 Mar;14(4):505-13. doi: 10.1517/14656566.2013.770473. Epub 2013 Feb 13. PMID: 23406528).

In the context of mTOR, several attempts for therapeutic combination approaches are known. Reita D, et al. (in: Synergistic Anti-Tumor Effect of mTOR Inhibitors with Irinotecan on Colon Cancer Cells. Cancers (Basel). 2019 Oct 17;11(10):1581. doi: 10.3390/cancers11101581. PMID: 31627299; PMCID: PMC6826690) describe that in vivo, irinotecan and AZD2014 combination drastically reduced ectopic patient-derived colon tumor growth and this combination was more potent than Folfox or Folfiri. Finally, the combination totally inhibited liver and lung metastases developed from orthotopic implantation of SW480 cells. Thus, the use of mTOR catalytic inhibitors, in association with other chemotherapeutic agents like irinotecan at low doses, potentially is a hope for colon cancer treatment.

McRee AJ, et al. (in: A phase I trial of everolimus in combination with 5-FU/LV, mFOLFOX6 and mFOLFOX6 plus panitumumab in patients with refractory solid tumors. Cancer Chemother Pharmacol. 2014 Jul;74(1):117-23. doi: 10.1007/s00280-014-2474-0. Epub 2014 May 13. PMID: 24819684) report a phase I study that investigated the safety, dose-limiting toxicity, and efficacy in three cohorts all treated with the mTOR inhibitor everolimus that was delivered (1) in combination with 5-fluorouracil with leucovorin (5-FU/LV), (2) with mFOLFOX6 (5-FU/LV + oxaliplatin), and (3) with mFOLFOX6 + panitumumab in patients with refractory solid tumors. Among the 24 patients enrolled with refractory metastatic colorectal cancer, the median time on treatment was 2.7 months with 45 % of patients remaining on treatment with stable disease for at least 3 months. They found that while a regimen of everolimus in addition to 5-FU/LV and mFOLFOX6 appears safe and tolerable, the further addition of panitumumab resulted in an unacceptable level of toxicity that cannot be recommended for further study. Further investigation were warranted to better elucidate the role which mTOR inhibitors play in patients with refractory solid tumors, with a specific focus on mCRC as a potential for the combination of this targeted and cytotoxic therapy in future studies.

Li, S., et al. (in: Combination of rapamycin and garlic-derived S-allylmercaptocysteine induces colon cancer cell apoptosis and suppresses tumor growth in xenograft nude mice through autophagy/p62/Nrf2 pathway. Oncology Reports, 38, 1637-1644. (2017). https://doi.org/10.3892/or.2017.5849) describe that the natural plant-derived product S-allylmercapto-cysteine (SAMC) has been studied in cancer therapy as a single and combination chemotherapeutic agent. The combination use of SAMC and rapamycin, the mTOR inhibitor with anticancer ability with limited efficacy due to drug resistance, was studies in order to explore the underlying mechanisms. They combined rapamycin and SAMC for colorectal cancer treatment in the HCT-116 cancer cells and a xenograft murine model. The in vivo study was established by xenografting HCT-116 cells in BALB/c nude mice. It was found that the combination therapy had enhanced tumor-suppressing ability with the upregulation of the Bax/Bcl-2 ratio as a consequence of activated apoptosis, inhibition of autophagic activity and prevention of Akt phosphorylation. The combination of SAMC and rapamycin enhanced the anticancer ability, which could be used for the treatment of colorectal cancer. The underling mechanism of autophagy/p62/Nrf2 pathway discovered may provide a new direction for drug development, especially for traditional Chinese medicines.

Chao TH, et al. (in: The synergistic effect of rapamycin combined with 5-fluorouracil in BALB/cByJNarl mice bearing CT-26 tumor cells. Anticancer Res. 2014 Jul;34(7):3329-35. PMID: 24982337) present the antitumor effect of rapamycin, an inhibitor of mammalian target of rapamycin (mTOR) signaling, combined with 5-fluorouracil treatment on CT-26 colorectal adenocarcinoma cells implanted into BALB/c mice. Rapamycin combined with 5-fluorouracil significantly reduced tumor size, suppressed expression of B-cell lymphoma 2, increased tumor apoptosis, and inhibited mTOR signaling activity by de-phosphorylation of S6K. Rapamycin combined with 5-fluorouracil treatment had a synergistic tumor-inhibition effect. Future research on rapamycin was said to be required to develop new therapeutic strategies.

Similarly, Chen YQ, et al. (in: Delivery of Rapamycin by Liposomes Synergistically Enhances the Chemotherapy Effect of 5-Fluorouracil on Colorectal Cancer. Int J Nanomedicine. 2021 Jan 12;16:269-281. doi: 10.2147/IJN.S270939. PMID: 33469286) prepared rapamycin liposomes using the ethanol injection method. The cellular uptake and biodistribution were detected by LC-MS and in vivo imaging system. MTT assay, transwell migration experiment, flow cytometry, and Western blot analysis evaluated the antitumor effect of rapamycin liposomes in vitro. Furthermore, HCT-116 tumor-bearing mice were used to assess the therapeutic efficacy of rapamycin liposomes in vivo. They demonstrated in vivo good antitumor efficacy of the rapamycin liposomes in HCT-116 xenograft mice. In addition, rapamycin liposomes and 5-FU are disclosed to synergistically improve the efficacy of colorectal cancer via the Akt/mTOR and P53 pathways.

P2X receptors (P2XRs) are a family of ATP-gated ionic channels that are expressed in numerous excitable and non-excitable cells. Despite the great advance on the structure and function of these receptors in the last decades, there is still lack of specific and potent antagonists for P2XRs subtypes, especially for the P2X4R. 5-(3-bromophenyl)-1,3-dihydro-2H-benzofuro[3,2-e]-1,4-diazepin-2-one (5-BDBD) was found to be a specific P2X4R antagonist, functioning on ATP-induced currents (see, for example, Coddou C, Sandoval R, Hevia MJ, Stojilkovic SS. Characterization of the antagonist actions of 5-BDBD at the rat P2X4 receptor. Neurosci Lett. 2019;690:219-224. doi: 10.1016/j.neulet.2018.10.047).

WO 2017/070660A1 discloses methods of inhibiting cancer cells by exposing the cancer cells to a purinergic receptor antagonist that targets one or more purinergic receptors of the cancer cells. The targeted purinergic receptors can include P2 purinergic receptors, such as P2X purinergic receptor subtypes (e.g., P2X3, P2X4, or P2X5). In some embodiments, the inhibited cancer cells are associated with hepatocellular carcinoma.

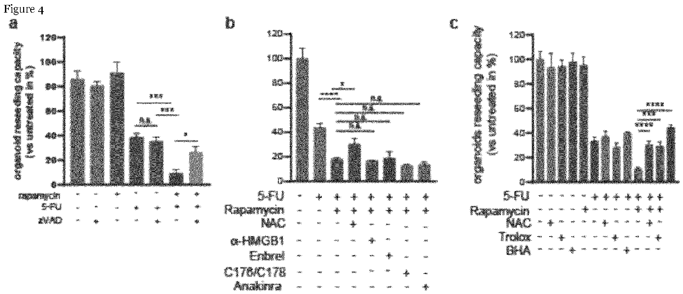

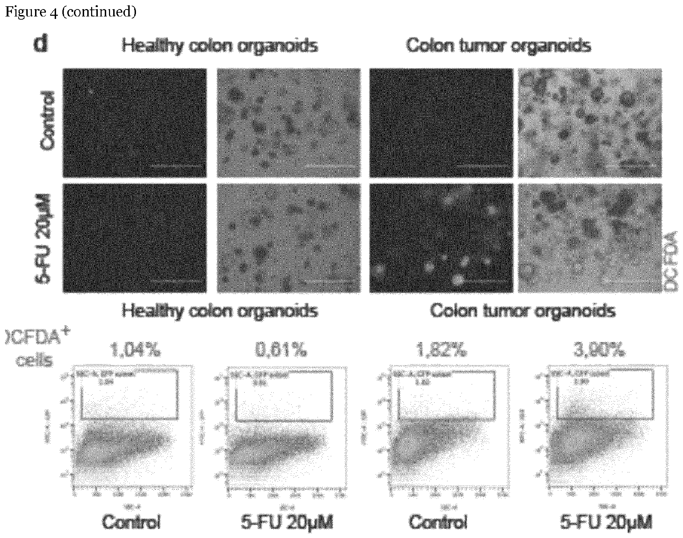

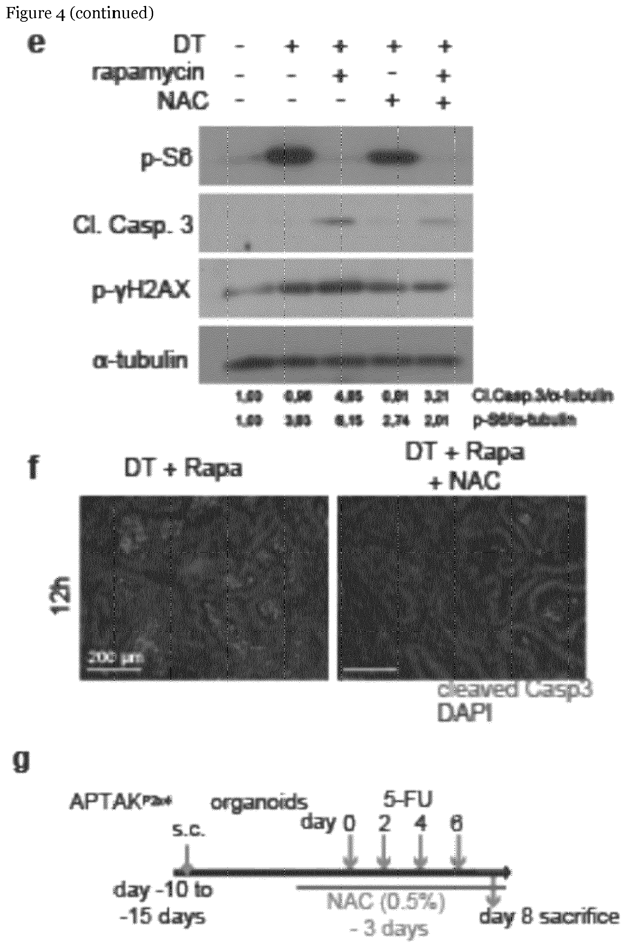

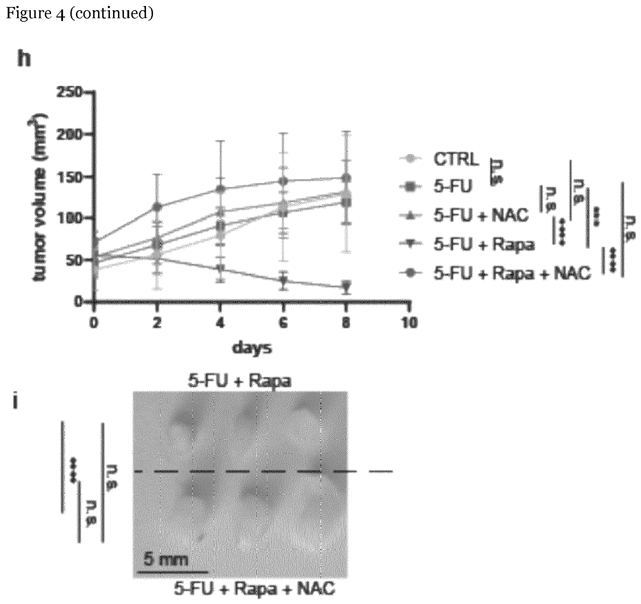

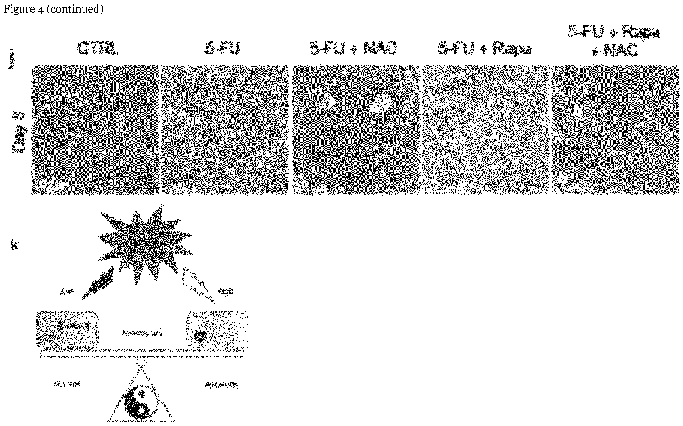

WO 2013/139940 relates to an inhibitor of the P2Y2 receptor or an inhibitor of the P2Y2 receptor signaling pathway for use in preventing the metastasis of tumours or as a lead compound for developing a drug for preventing the metastasis of tumours. Figure 4 shows the effect of the P2X4 receptor antagonist 5-BDBD (1 µM) on platelet-stimulated transendothelial migration of B16 cells.

He et al. (in: He J, Zhou Y, Arredondo Carrera HM, et al. Inhibiting the P2X4 Receptor Suppresses Prostate Cancer Growth In Vitro and In Vivo, Suggesting a Potential Clinical Target. Cells. 2020;9(11):2511. Published 2020 Nov 20. doi:10.3390/cells9112511) describe the effect of inhibiting P2X4R on prostate cancer (PCa) (PC3 and C4-2B4 cells) viability, proliferation, migration, invasion, and apoptosis were examined using the selective P2XR4 antagonists 5-BDBD and PSB-12062. The results demonstrated that inhibiting P2X4R impaired the growth and mobility of PCa cells but not apoptosis. They suggest that P2X4R has a role in enhancing PCa tumour formation and is a clinically targetable candidate for which inhibitors are already available and have the potential to suppress disease progression.

Campos-Contreras ADR, Díaz-Muñoz M, and Vázquez-Cuevas FG. (in: Purinergic Signaling in the Hallmarks of Cancer. Cells. 2020;9(7):1612. Published 2020 Jul 3. doi:10.3390/cells9071612) review the purinergic system, a signaling pathway formed by nucleotides/nucleosides (mainly adenosine triphosphate (ATP), adenosine (ADO) and uridine triphosphate (UTP)) with their corresponding membrane receptors and defined transduction mechanisms. The dynamic equilibrium between ATP and ADO, which is accomplished by the presence and regulation of a set of ectonucleotidases, defines the pro-carcinogenic or anti-cancerous final outline in tumors and cancer cell lines. So far, the purinergic system has been recognized as a potential therapeutic target in cancerous and tumoral ailments.

Finally, Di Virgilio, F., et al. (in : Extracellular ATP and P2 purinergic signalling in the tumour microenvironment. Nat Rev Cancer 18, 601-618 (2018). https://doi.org/10.1038/s41568-018-0037-0) disclose that one of the most potent immunosuppressive factors is adenosine, which is generated in the tumour microenvironment owing to degradation of extracellular ATP. Accruing evidence over the past few years shows that ATP is one of the major biochemical constituents of the tumour microenvironment, where it acts at P2 purinergic receptors expressed on both tumour and host cells. Stimulation of P2 receptors has different effects depending on the extracellular ATP concentration, the P2 receptor subtype engaged and the target cell type. Among P2 receptors, the P2X purinergic receptor 7 (P2X7R) subtype appears to be a main player in host-tumour cell interactions. Preclinical studies in several tumour models have shown that P2X7R targeting is potentially a very effective anticancer treatment, and many pharmaceutical companies have now developed potent and selective small molecule inhibitors of P2X7R. In this review, they report on the multiple mechanisms by which extracellular ATP shapes the tumour microenvironment and how its stimulation of host and tumour cell P2 receptors contributes to determining tumour fate.

Thus, it is an object of the invention to provide new and more effective combination treatment approaches for the prevention and/or treatment of cancerous diseases, in particular solid tumours as well as metastases thereof, such as for example in colorectal cancer. Other objects and advantages will become apparent to the person of skill when studying the present description of the present invention.

Generally, and by way of brief description, the main aspects of the present invention can be described as follows: In a first aspect of the present invention the above object is solved by providing an effective amount of an inhibitor of the P2X4 receptor or an inhibitor of the P2X4 receptor signaling pathway in combination with an effective amount of a cell death inducing chemotherapeutic drug and/or a cell death inducing therapy for use in the prevention and/or treatment of a solid tumor or metastases thereof in a subject. Preferably, therefore, said treatment and/or prevention comprises the inhibition of the ATP-dependent P2X4 receptor- and/or P2X4 receptor signaling pathway-mediated tumor escape mechanism(s).

In a second aspect of the present invention the above object is solved by a method of preventing and/or treating a solid tumor or metastases thereof in a subject in need thereof, the method comprising the concomitant or sequential administration of (i) an effective amount of an inhibitor of the P2X4 receptor or an inhibitor of the P2X4 receptor signaling pathway, and (ii) an effective amount of a cell death inducing chemotherapeutic drug and/or a cell death inducing therapy to said subject.

Preferably, said treatment and/or prevention comprises the inhibition of the ATP-dependent P2X4 receptor- and/or P2X4 receptor signaling pathway-mediated tumor escape mechanism(s).

In the following, the elements of the invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect of the present invention the above object is solved by providing an effective amount of an inhibitor of the P2X4 receptor or an inhibitor of the P2X4 receptor signaling pathway in combination with an effective amount of a fluoropyrimidine drug for use in the prevention and/or treatment of a solid tumor or metastases thereof in a subj ect.

In context of the herein disclosed invention, the terms "inhibitor of the P2X4 receptor" and "inhibitor of the P2X4 receptor signaling pathway", shall be understood to refer to any compound or means that when brought into contact with a cell inhibit the biological function of the P2X4 receptor, in particular the purinergic signaling thereof in the context of a tumor or tumor environment. Quite a few compounds have been described to have an activity as inhibitor of the P2X4 receptor and/or inhibitor of the P2X4 receptor signaling pathway, and such compounds, known to the skilled person, shall be encompassed by the present invention. In preferred embodiments, the inhibitor of the P2X4 receptor is a specific inhibitor of the P2X4 receptor, i.e. does not bind or does not substantially bind to other P2X or P2Y receptors, and is preferably selected from the group of PPADS, Suramin, KN-62, TNP-ATP, Brilliant Blue G, 5-BDBD, BX-430, Carbamazepine der., PSB-12054, PSB-12062, PSB-15417, NP-1815-PX, NC-2600, UoS14919, Paroxetine, Duloxetine, BAY-1797, IgG#151-LO, an antibody or a fragment or derivative thereof, a siRNA, a shRNA, a miRNA, a ribozyme, an aptamer, an antisense nucleic acid molecule, a small molecule or a modified version of these inhibitors (see, for example, Bragança B, Correia-de-Sá P. Resolving the Ionotropic P2X4 Receptor Mystery Points Towards a New Therapeutic Target for Cardiovascular Diseases. Int J Mol Sci. 2020 Jul 15;21(14):5005. doi: 10.3390/ijms21145005. PMID: 32679900; PMCID: PMC7404342). Preferred is the inhibitor 5DBDB.

In context of the herein disclosed invention, the term "cell death inducing chemotherapeutic drug" or "cell death inducing therapy" shall mean any suitable drug or therapy (e.g. chemotherapy, irradiation, immunotherapy, apoptosis inducing therapy, etc...) that induces cellular death in cells, such as, for example, cancer cells. One preferred but exemplary example in the context of the present invention is 5-FU, a fluoropyrimidine drug. Other examples are necrotic cell supernatants; cationic amphiphilic drugs (CAD); classical anticancer agents, including anthracyclines, antimetabolites, and platinum drugs; topoisomerase inhibitors, e.g. camptothecin; PDE3A inhibitors, such as anagrelide, either alone or with cell death-inducing cytokines; death-inducing cytokines, such as IFN-α, IFN-γ, TNF-α, or TRAIL; taxanes, paclitaxel, and fluorinated taxanes, such as SB-T-12851, SB-T-12852, SB-T-12853, and SB-T-12854. Other suitable molecules and compounds induce apoptosis (as, for example, mentioned in Pfeffer CM, Singh ATK. Apoptosis: A Target for Anticancer Therapy. Int J Mol Sci. 2018;19(2):448. Published 2018 Feb 2. doi:10.3390/ijms19020448).

In context of the herein disclosed invention, the term "fluoropyrimidine drug" shall be understood to refer to any compound having a comparable and/or substantially identical chemical structure and pharmaceutical activity to the anti-cancer drug 5-fluorouracil (5-FU). Preferably, said fluoropyrimidine drug (FP) is selected from the group of 5-fluorouracil, tegafur, capecitabine, and doxifluridine, preferably 5-fluorouracil (5-FU).

In the context of the present invention, the term "effective amount" shall mean the amount of drug(s) in a composition as administered to a subject that produces a (desired) biological response. This is generally defined by the range between the minimum effective dose (MED) and the maximum tolerated dose (MTD). The MED is defined as the lowest dose level of a pharmaceutical product that provides a clinically significant response in average efficacy, which is also statistically significantly superior to the response provided by the placebo. Similarly, the MTD is the highest possible but still tolerable dose level with respect to a pre-specified clinical limiting toxicity. In general, these limits refer to the average patient population. Thus, the person of skill will be readily able to identify such amount (see also below).

As explained herein, the present invention is based on the surprising finding that tumor cells - when killed during chemo- and/or toxin therapy - release messenger compounds, in particular ATP, into the tumor environment that improve survival of the neighboring tumour cells based on the functions of mTOR and P2x4. Therefore, the preventive and/or therapeutic treatment in context of the invention is based on a combinatorial approach of inhibiting the ATP-dependent P2X4 receptor- and/or P2X4 receptor signaling pathway-mediated tumor escape mechanism(s) together with the toxicity of the amount of a cell death inducing chemotherapeutic drug and/or a cell death inducing therapy. This combination (optionally with other agents as disclosed herein), as exemplified by the preferred combination of 5-FU and 5DBDB, effectively blocks this escape-mechanism, and with it a further growth of the tumor and/or the formation of metastases or a relapse. In context of the herein disclosed invention cells surrounding a cell or tissue involved with the proliferative disorder are cells of the microenvironment of the diseased tissue, such a tumour microenvironment.

In general, any suitable inhibitor of the P2X4 receptor or inhibitor of the P2X4 receptor signaling pathway can be used in the context of the present invention, as long as it can be combined (either provided together with, jointly, or separately as long as functioning together in the subject) with the cell death inducing chemotherapeutic drug and/or a cell death inducing therapy. Preferred is an inhibitor of the P2X4 receptor that is a specific inhibitor of the P2X4 receptor or a specific inhibitor of the components of the P2X4 receptor signaling pathway. In the context of the present invention, "specific inhibitor" shall mean that the inhibitor compound does not bind or does not substantially bind to other receptors and/or receptor signaling pathways in the targeted cell(s). In particular, a specific inhibitor is preferred, if it shows more than 2-fold, more than 3-fold, more than 10-fold, more than 35-fold, more than 50-fold, more than 100-fold, more than 1000-fold, or even more than 10000-fold stronger binding to the human P2X4 receptor or to components of the P2X4 receptor signaling pathway, than to other members of the P2X and/or P2Y family and their signaling pathways.

Preferably, the inhibitor is selected from the group consisting of PPADS, Suramin, KN-62, TNP-ATP, Brilliant Blue G, 5-BDBD, BX-430, Carbamazepine der., PSB-12054, PSB-12062, PSB-15417, NP-1815-PX, NC-2600, UoS14919, Paroxetine, Duloxetine, BAY-1797, IgG#151-LO, an antibody or a fragment or derivative thereof, a siRNA, a shRNA, a miRNA, a ribozyme, an aptamer, an antisense nucleic acid molecule, a small molecule and a chemically modified version of these inhibitors, and is preferably selected from 5DBDB. Any inhibitor of a therapeutic target defined in the disclosed invention, i.e. P2X4 receptor or its signalling pathway, such as a compound or composition selected from a polypeptide, peptide, glycoprotein, a peptidomimetic, an antibody or antibody-like molecule (such as an intra-body); a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a genetic construct for targeted gene editing, such as a CRISPR/Cas9 construct and/or guide RNA/DNA (gRNA/gDNA) and/or tracrRNA; a hetero-bi-functional compound (such as a PROTAC or a HyT molecule); a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, or small cell-permeable molecules, may be used in the context of the present invention.

In general, any suitable cell death inducing chemotherapeutic drug and/or a cell death inducing therapy can be used in the context of the present invention, as long as it can be combined (either provided together with, jointly, or separately as long as functioning together in the subject) with the above inhibitor of the P2X4 receptor inhibitor of the components of the P2X4 receptor signaling pathway. Preferably, necrotic cell supernatants; cationic amphiphilic drugs (CAD); classical anticancer agents, including anthracyclines, antimetabolites, and platinum drugs; topoisomerase inhibitors, e.g. camptothecin; PDE3A inhibitors, such as anagrelide, either alone or with cell death-inducing cytokines; death-inducing cytokines, such as IFN-α, IFN-γ, TNF-α, or TRAIL; taxanes, paclitaxel, and fluorinated taxanes, such as SB-T-12851, SB-T-12852, SB-T-12853, and SB-T-12854 may be used as drugs, when a fluoropyrimidine drug (FP) is used it is preferably selected from the group of 5-fluorouracil, tegafur, capecitabine, and doxifluridine, and other FP derivatives, more preferably 5-fluorouracil (5-FU).

Preferred is the use of the inhibitor of the P2X4 receptor or inhibitor of the P2X4 receptor signaling pathway in combination with the cell death inducing chemotherapeutic drug and/or a cell death inducing therapy according to the present invention, wherein said treatment and/or prevention further comprises a prior and/or concomitant anti-cancer chemotherapy, comprising for example cellular toxins, Lgr5 inhibitors, Lgr5+ cancer cell ablation, mTOR inhibitors, and IMPDH inhibitors. In general, any suitable anti-cancer chemotherapy can be used in the context of the present invention, as long as it can be combined (either provided together with, jointly, or separately as long as functioning together in the subject) with the above combination of the present invention, which effectively blocks the cancer escape-mechanism as disclosed herein, and with it a further growth of the tumor and/or the formation of metastases or a relapse.

In context of the invention the subject treatable by the therapies of the invention is preferably distinguished as having a tumour that progressed, in particular relapsed, recurred or did not respond to, prior chemo-, toxin-, and/or radiotherapy. For example the tumour disease is a reoccurring or relapsing tumour disease (metastases).

As mentioned above the therapy and/or the additional treatment may comprise at least one or more (cell death inducing) cancer therapies including chemotherapy, a radiation therapy, or immune therapy. The treatment regimen and schedule may vary depending on the type(s) of preconditioning and cancer therapies. For example, a chemotherapy or a radiation therapy may be administered to a patient at least 1 day, 3 days, 5 days, 7 days after completing preconditioning of the tumour. In another example, in some embodiments, a cell-based immune therapy may be administered to a patient at least 1 day, 3 days, 5 days, 7 days before starting the inventive therapy. In still other embodiments, the additional therapy may be administered to the patient during the inventive therapy (e.g., at least 12 hours, at least 1 day, at least 3 days after beginning of preconditioning, etc.). Thus, the treatment of the invention may further comprise the administration of at least one additional anti-proliferative therapeutic such as a chemotherapeutic agent.

Preferred is the inhibitor of the P2X4 receptor or inhibitor of the P2X4 receptor signaling pathway in combination with the fluoropyrimidine drug for use according to the present invnetion, wherein said combination is provided simultaneously or separately, as combined and/or separate dosage forms.

As used herein, "cancer" can include the term "solid tumour." As used herein, the term "solid tumour" refers to those conditions, such as cancer, that form an abnormal tumour mass, such as sarcomas, carcinomas, and lymphomas. Examples of solid tumours or metastases thereof are selected from prostate cancer, pancreatic cancer, breast cancer, gastric cancer, liver cancer, brain cancer, lung cancer, kidney cancer, and colorectal cancer, and metastases thereof, in particular colorectal cancer, and metastases thereof, the solid tumour disease can be, for example, an adenocarcinoma, squamous cell carcinoma, large cell carcinoma, and the like.

In certain embodiments, the cancer can be, for example, primary or secondary (i.e. metastases) esophageal cancer, gastroesophageal junction cancer, gastroesophageal adenocarcinoma, gastric cancer, chondrosarcoma, colorectal adenocarcinoma, breast cancer, ovarian cancer, head and neck cancer, melanoma, gastric adenocarcinoma, lung cancer, pancreatic cancer, renal cell carcinoma, hepatocellular carcinoma, cervical cancer, brain tumour, multiple myeloma, leukaemia, lymphoma, prostate cancer, cholangiocarcinoma, endometrial cancer, small bowel adenocarcinoma, uterine sarcoma, or adrenocorticoid carcinoma. In certain embodiments, the cancer can be, for example, esophageal cancer, gastroesophageal junction cancer, gastroesophageal adenocarcinoma, colorectal adenocarcinoma, breast cancer, ovarian cancer, head and neck cancer, melanoma, gastric adenocarcinoma, lung cancer, pancreatic cancer, renal cell carcinoma, hepatocellular carcinoma, cervical cancer, brain tumour, multiple myeloma, leukaemia, lymphoma, prostate cancer, cholangiocarcinoma, endometrial cancer, small bowel adenocarcinoma, uterine sarcoma, or adrenocorticoid carcinoma. In certain embodiments, the cancer can be, for example, colorectal cancer. In certain embodiments, the cancer can be, for example, colorectal adenocarcinoma. In certain embodiments, the cancer can be, for example, small bowel adenocarcinoma. In certain embodiments, the cancer can be, for example, hepatocellular carcinoma. In certain embodiments, the cancer can be, for example, head and neck cancer. In certain embodiments, the cancer can be, for example, renal cell carcinoma. In certain embodiments, the cancer can be, for example, ovarian cancer. In certain embodiments, the cancer can be, for example, prostate cancer. In certain embodiments, the cancer can be, for example, lung cancer. In certain embodiments, the cancer can be, for example, uterine sarcoma. In certain embodiments, the cancer can be, for example, esophageal cancer. In certain embodiments, the cancer can be, for example, endometrial cancer. In certain embodiments, the cancer can be, for example, cholangiocarcinoma. In certain embodiments, each of the cancers can be, for example, unresectable, advanced, refractory, recurrent, or metastatic. A preferred cancer is a solid cancer, in particular hepatocellular carcinoma or colorectal cancer.

The herein disclosed invention in the various aspects and embodiments suggest a combinatorial treatment regime for cancer therapy. In such aspects and embodiments combination of the following agents is most preferred; 5-fluorouracil (as cell-death antiproliferative agent), and 5DBDB (as inhibitor of the P2X4 receptor).

In a preferred aspect of the present invention, the above object is solved by a method for screening for an effective, preferably synergistic, combination of an inhibitor of the P2X4 receptor or inhibitor of the P2X4 receptor signaling pathway and cell death inducing chemotherapeutic drug and/or a cell death inducing therapy, comprising contacting a solid tumor or metastases thereof with at least one of a candidate inhibitor of the P2X4 receptor or candidate inhibitor of the P2X4 receptor signaling pathway in combination with a candidate cell death inducing chemotherapeutic drug and/or a cell death inducing therapy with a solid tumor, and detecting at least one of shrinking of said tumor, increase of apoptosis of said tumor cells, death of tumor cells, and reduction of metastases stemming from said tumor cells, and thereby identifying an effective, preferably synergistic, combination of candidate compounds or therapy. Candidate compounds can be selected as described above.

Preferably, said screening is performed in cell culture, such as, for example in 3D culture using organoids of tumor cells.

More preferred is a screening method wherein said method further comprises a prior and/or concomitant addition of compounds for anti-cancer chemotherapy, comprising for example cellular toxins, Lgr5 inhibitors, Lgr5+ cancer cell ablation, mTOR inhibitors, and IMPDH inhibitors. More preferred is a method according to the invention, wherein said combination is provided simultaneously or separately, as combined and/or separate dosage forms.

More preferred is a method according to the invention, wherein said screening comprises the detection of the inhibition of the ATP-dependent P2X4 receptor- and/or P2X4 receptor signaling pathway-mediated tumor escape mechanism.

The combination as identified, preferably the synergistic combination can be formulated into an anti-cancer formulation as disclosed herein, and can be used in the context of the present invention.

In another preferred aspect of the present invention, the above object is solved by a method of preventing and/or treating a solid tumor or metastases thereof in a subject in need thereof, the method comprising the concomitant or sequential administration of (i) an effective amount of an inhibitor of the P2X4 receptor or an inhibitor of the P2X4 receptor signaling pathway, and (ii) an effective amount of a cell death inducing chemotherapeutic drug and/or a cell death inducing therapy to said subject.

Compounds that are suitable and/or suitable for therapy are as disclosed above. To be used in therapy, the compounds and compositions for use in the invention may be formulated into a pharmaceutical composition appropriate to facilitate administration to animals or humans. The term "pharmaceutical composition" means a mixture of substances including a therapeutically active substance (such as an agonist or antagonist of the invention) for pharmaceutical use as disclosed herein. Accordingly, in an additional aspect, the invention relates to the use of a pharmaceutical composition comprising one or more compounds for use in the invention, i.e. (i) an effective amount of an inhibitor of the P2X4 receptor or an inhibitor of the P2X4 receptor signaling pathway, and (ii) an effective amount of a cell death inducing chemotherapeutic drug and/or a cell death inducing therapy, and a pharmaceutically acceptable carrier, stabiliser and/or excipient.

The pharmaceutical composition for use in the invention may comprise the combination therapeutics in accordance with the herein disclosed treatment regimens, or alternatively, the invention may comprise a combination of pharmaceutical compositions, wherein each pharmaceutical composition comprises one single component of the combinatorial therapeutics of the invention, such as preferably one composition comprising the inhibitor and a and a pharmaceutically acceptable carrier, stabiliser and/or excipient; another pharmaceutical composition comprising the fluoropyrimidine drug and a pharmaceutically acceptable carrier, stabiliser and/or excipient. The compositions of the invention in this embodiment may be combined into one therapeutic kit.

By way of example, the pharmaceutical composition for use in the invention may comprise between 0.1% and 100% (w/w) active ingredient (for example, inhibitor of the P2X4 receptor or an inhibitor of the P2X4 receptor signaling pathway and/or a cell death inducing chemotherapeutic drug and/or a cell death inducing therapy), such as about 0.5%, 1%, 2%, 3%, 4%, 5%, 6%, 8% 10%, 15%, 20%, 25%, 30%, 40%, 50%, 60%, 70%, 80%, 90%, 95%, 96%, 97%, 98% or 99%, preferably between about 1% and about 20%, between about 10% and 50% or between about 40% and 90%.

As used herein the term "pharmaceutically acceptable" excipient, stabiliser or carrier is intended to include any and all solvents, solubilisers, fillers, stabilisers, binders, absorbents, bases, buffering agents, lubricants, controlled release vehicles, diluents, emulsifying agents, humectants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The pharmaceutical composition of (or for use with) the invention is, typically, formulated to be compatible with its intended route of administration. Examples of routes of administration include oral, parenteral, e.g., intrathecal, intra-arterial, intravenous, intradermal, subcutaneous, oral, transdermal (topical) and transmucosal administration. Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Kolliphor^{®} EL (formerly Cremophor EL^{™}; BASF, Parsippany, N.J.) or phosphate buffered saline (PBS). In all cases, the injectable composition should, typically, be sterile and be fluid to the extent that easy syringability exists. It should, typically, be stable under the conditions of manufacture and storage and be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the requited particle size in the case of dispersion and by the use of surfactants. Lipid particles may be included as well (see above for rapamycin). Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the injectable compositions can be brought about by including in the composition an agent that delays absorption, for example, aluminium monostearate and gelatin.

In some embodiments, the pharmaceutical composition comprising an inhibitor of the P2X4 receptor or an inhibitor of the P2X4 receptor signaling pathway is in unit dose form of between 10 and 1000 mg inhibitor. In some embodiments, the pharmaceutical composition comprising inhibitor is in unit dose form of between 10 and 200 mg, or between 200 and 400 mg, or even between 400 and 600 mg, or between 600 and 800 mg of an inhibitor of the P2X4 receptor or an inhibitor of the P2X4 receptor signaling pathway. Similar doses are used for the fluoropyrimidine drug in accordance with the invention.

Exemplary unit dosage forms for pharmaceutical compositions comprising an inhibitor of the P2X4 receptor or an inhibitor of the P2X4 receptor signaling pathway and/or cell death inducing chemotherapeutic drug(s) are tablets, capsules (eg as powder, granules, microtablets or micropellets), suspensions or as single-use pre-loaded syringes. In certain embodiments, kits are provided for producing a single-dose administration unit. The kit can contain both a first container having a dried active ingredient and a second container having an aqueous formulation. Alternatively, the kit can contain single and multi-chambered pre-loaded syringes.

In accordance with all aspects and embodiments of the medical uses and methods of treatment provided herein, the effective amount administered at least once to a subject in need of treatment with a compound or composition for use in the invention is, typically, between about 0.01mg/kg and about 100 mg/kg per administration, such as between about 1mg/kg and about 10mg/kg per administration. In some embodiments, the effective amount administered at least once to said subject of a compound or composition of the invention is between about 0.01 mg/kg and about 0. 1mg/kg per administration, between about 0.1 mg/kg and about 1 mg/kg per administration, between about 1mg/kg and about 5mg/kg per administration, between about 5mg/kg and about 10mg/kg per administration, between about 10mg/kg and about 50mg/kg per administration, or between about 50mg/kg and about 100mg/kg per administration.

For the prevention or the treatment of the solid tumors and/or relapses and/or metastases, the appropriate dosage of a compound or composition for use in the invention (or a pharmaceutical composition comprised thereof) will depend on the type of disease to be treated, the severity and course of the disease, whether the compound or composition of the invention and/or pharmaceutical composition is administered for preventive or therapeutic purposes, previous therapy, the patient's clinical history, age, size/weight and response to the compound or composition of the invention and/or pharmaceutical composition, and the discretion of the attending physician. The compound or composition of the invention and/or pharmaceutical composition is suitably administered to the patient at one time or over a series of treatments. If such compound or composition of the invention and/or pharmaceutical composition is administered over a series of treatments, the total number of administrations for a given course of treatment may consist of a total of about 2, 3, 4, 5, 6, 7, 8, 9, 10 or more than about 10 treatments. For example, a treatment may be given once every day (or 2, 3 or 4 times a day) for a week, a month or even several months. In certain embodiments, the course of treatment may continue indefinitely, or until no more needed.

Preferred is the method according to the present invention, wherein said inhibitor of the P2X4 receptor is a specific inhibitor of the P2X4 receptor, and is preferably selected from the group of PPADS, Suramin, KN-62, TNP-ATP, Brilliant Blue G, 5-BDBD, BX-430, Carbamazepine der., PSB-12054, PSB-12062, PSB-15417, NP-1815-PX, NC-2600, UoS14919, Paroxetine, Duloxetine, BAY-1797, IgG#151-LO, an antibody or a fragment or derivative thereof, a siRNA, a shRNA, a miRNA, a ribozyme, an aptamer, an antisense nucleic acid molecule, a small molecule or a modified version of these inhibitors, preferably 5DBDB. Regarding "specific" and other characteristics, see above.

Preferred is the method according to the present invention, wherein said cell death inducing chemotherapeutic drug is a fluoropyrimidine drug (FP), and is selected from the group of 5-fluorouracil, tegafur, capecitabine, and doxifluridine, preferably 5-fluorouracil (5-FU). Other preferred examples are necrotic cell supernatants; cationic amphiphilic drugs (CAD); classical anticancer agents, including anthracyclines, antimetabolites, and platinum drugs; topoisomerase inhibitors, e.g. camptothecin; PDE3A inhibitors, such as anagrelide, either alone or with cell death-inducing cytokines; death-inducing cytokines, such as IFN-α, IFN-γ, TNF-α, or TRAIL; taxanes, paclitaxel, and fluorinated taxanes, such as SB-T-12851, SB-T-12852, SB-T-12853, and SB-T-12854.

Preferred is the method according to the present invention, wherein said treatment and/or prevention comprises the inhibition of the ATP-dependent P2X4 receptor- and/or P2X4 receptor signaling pathway-mediated tumor escape mechanism(s), and/or wherein said treatment and/or prevention further comprises a prior and/or concomitant anti-cancer chemotherapy, comprising for example cellular toxins, Lgr5 inhibitors, Lgr5+ cancer cell ablation, mTOR inhibitors, and IMPDH inhibitors, as also elaborated above..

As used herein, a "subject" includes all mammals, including without limitation humans, but also non-human primates such as cynomolgus monkeys. It also includes dogs, cats, horses, sheep, goats, cows, rabbits, pigs and rodents (such as mice and rats). It will be appreciated that a particularly preferred subject according to the invention is a human subject, such as a human suffering from (or at risk of suffering from) a disorder, disease or condition, for example a human patient.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein. Unless context dictates otherwise, the descriptions and definitions of the features set out above are not limited to any particular aspect or embodiment of the invention and apply equally to all aspects and embodiments which are described.

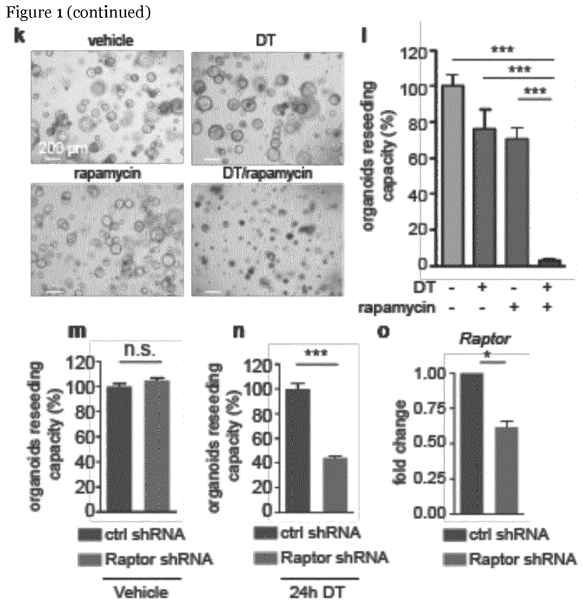

To functionally confirm that the engaged MAP kinases or mTOR activation were indeed responsible for survival of Lgr5+ cell-depleted organoids the inventors applied SP600125, PH797804, or rapamycin to inhibit JNK, p38, and mTOR, respectively, in the presence or absence of DT. While the two MAPK inhibitors did not affect tumor organoid survival combined application of DT and rapamycin led to substantial cell death within 24 hours selectively in DTR expressing tumor organoids (Fig. 1k). Moreover, re-seeding of DT/rapamycin co-treated organoids resulted in a markedly reduced capacity to form organoids, which was not observed when either compound was applied individually (Fig. 1l).

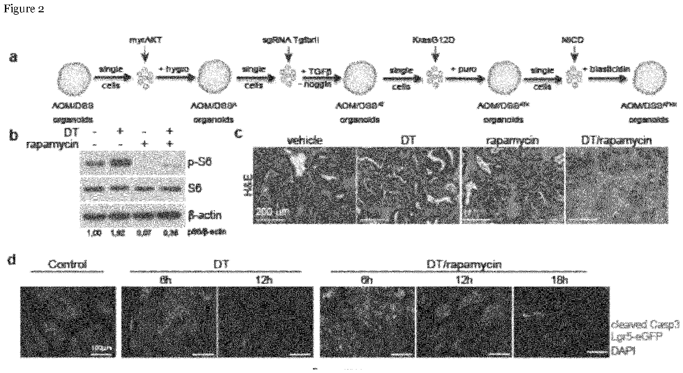

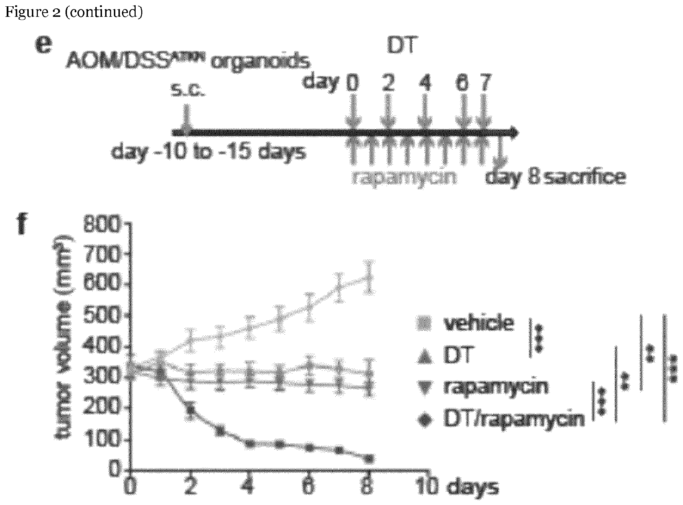

Treatment of Lgr5^{EGFPDTR(+)} tumor organoids and s.c. transplanted AOM/DSSATKN tumors with the IMPDH inhibitor mizoribine in combination with DT-mediated depletion of Lgr5+ cells resulted in profound organoid collapse *ex vivo* and markedly reduced viable proliferative of AOM/DSSATKN tumors without affecting S6-ribosomal protein phosphorylation. These data suggested that *de novo* purine synthesis was required downstream of mTOR activation for maintaining tumor integrity in the absence of Lgr5+ cells. When Lgr5+ cells were sufficiently ablated from the tumor, the inventors detected massive apoptosis in the tumor tissue of DT/rapamycin treated animals whereas almost no apoptosis was detectable in animals treated only with DT (Fig. 2d). As a consequence, prolonged rapamycin treatment plus Lgr5+ cell ablation caused marked tumor shrinkage of established tumors in contrast to single rapamycin or DT administration that individually applied led only to tumor stasis (Fig. 2e-g).

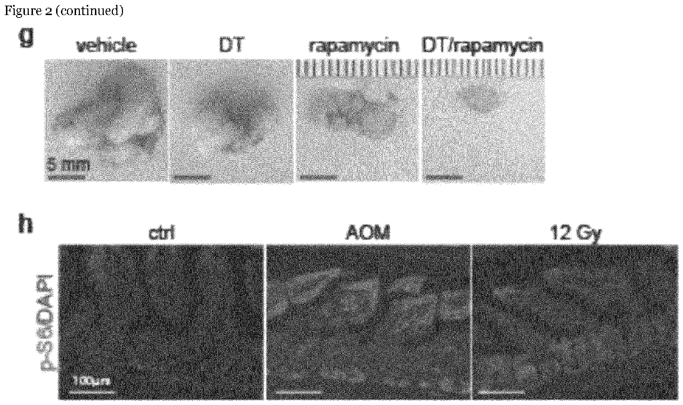

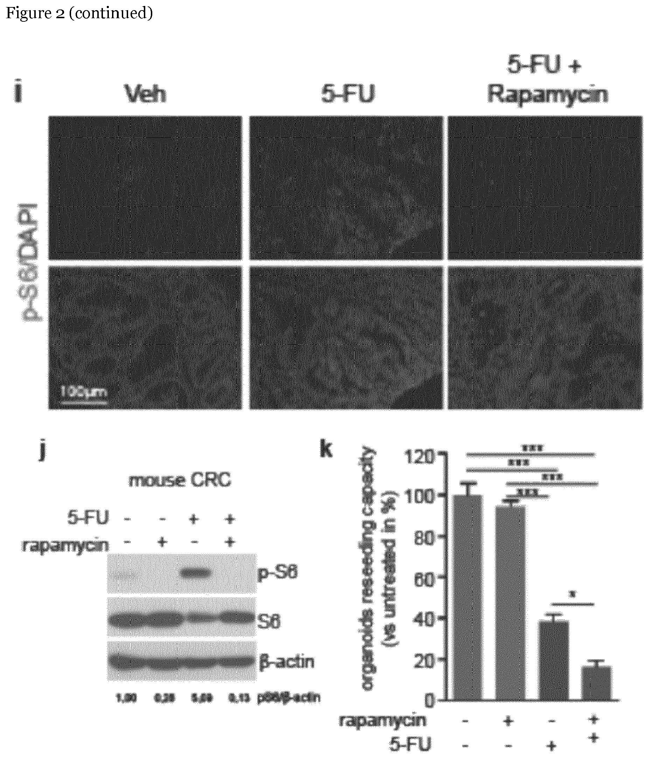

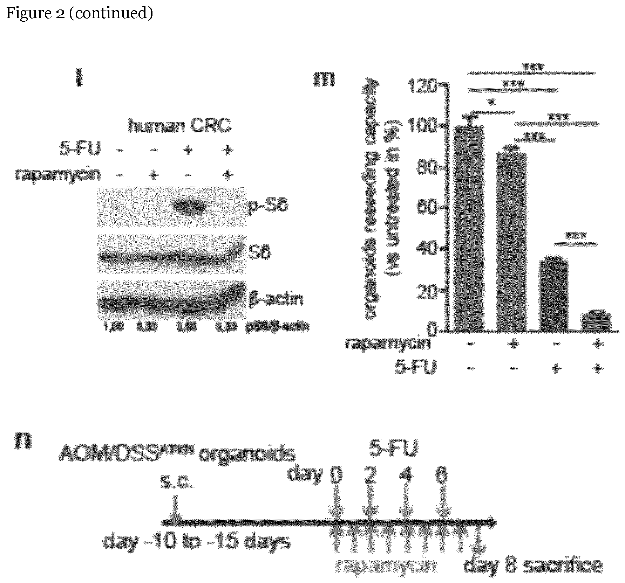

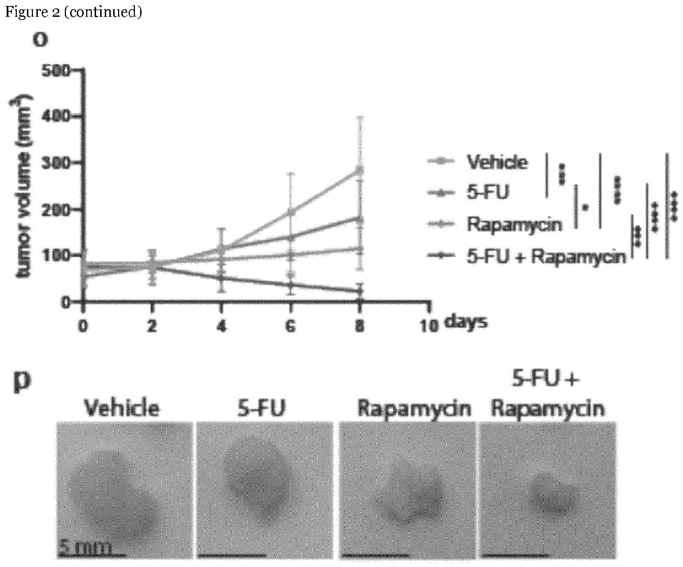

It was also found that combined mTOR inhibition and cell death induction can be employed therapeutically to enhance cytotoxic therapy response in tumors, and combination of rapamycin with 5-FU significantly suppressed reseeding capacity of tumor organoids (Fig. 2 k,m), and significantly reduced tumor growth of subcutaneously transplanted tumors when compared to the control or each treatment alone (Fig. 2n-p).

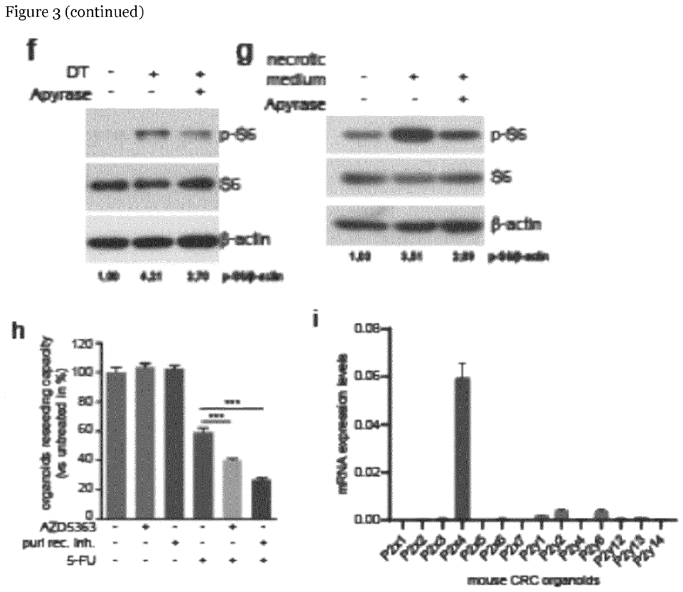

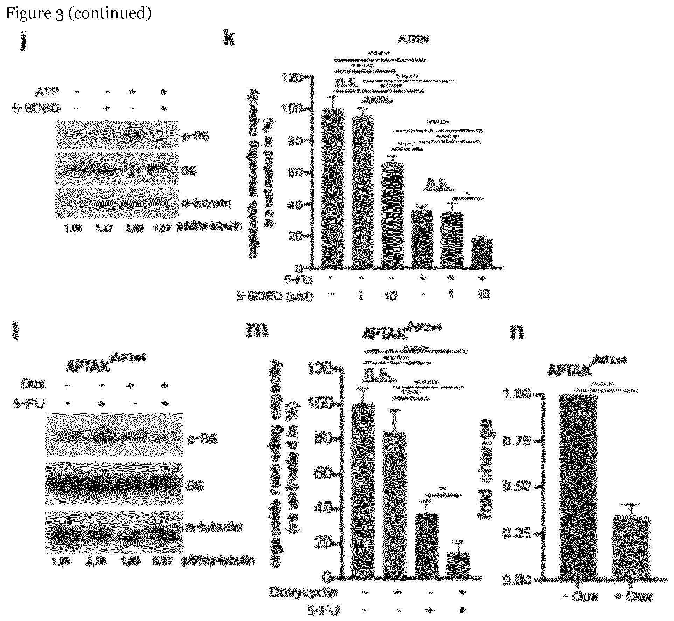

In confirmation of an important function of ATP for the paracrine activation of mTOR signaling by dying by Lgr5+ cells, the inventors were able to reduce the levels of S6 phosphorylation induced by DT treatment or necrotic supernatant by hydrolyzing the released ATP to AMP and inorganic phosphate using Apyrase (Fig. 3f,g). Blocking purinergic receptors of the P2 family using a combination of nonselective P2 receptor antagonists in combination with 5-FU phenocopied the effect of the 5-FU/rapamycin double treatment on organoid survival (Fig. 3h). P2x4 was remarkably higher expressed in murine tumor cells compared to all other P2 receptors (Fig. 3i), and chemical inhibition of P2x4 in combination with 5-FU treatment led to decreased organoid survival in AOM/DSS and APTAK tumor organoids when compared to each treatment alone (Fig. 3k). Similar results were found in genetic knockdowns of P2x4 in APTAK tumor organoids.

In conclusion, the inventors' experiments revealed how an increased ATP release from dying tumor cells (because of cell death inducing chemotherapeutic drugs and/or a cell death inducing therapy) activates mTOR survival signals in adjacenttumor cells in a P2x4 receptor dependent manner. This activation is essential for counteracting an increase of pro-apoptotic ROS signals in the tumor in order to help remaining tumor cells to escape cell death and to maintain tumor integrity (Fig. 4k). These findings open up a new strategy for an effective treatment and/or prevention of solid tumors, and metastases.

The present invention will now be described further in the following examples with reference to the accompanying Figures, nevertheless, without being limited thereto. For the purposes of the present invention, all references as cited herein are incorporated by reference in their entireties.

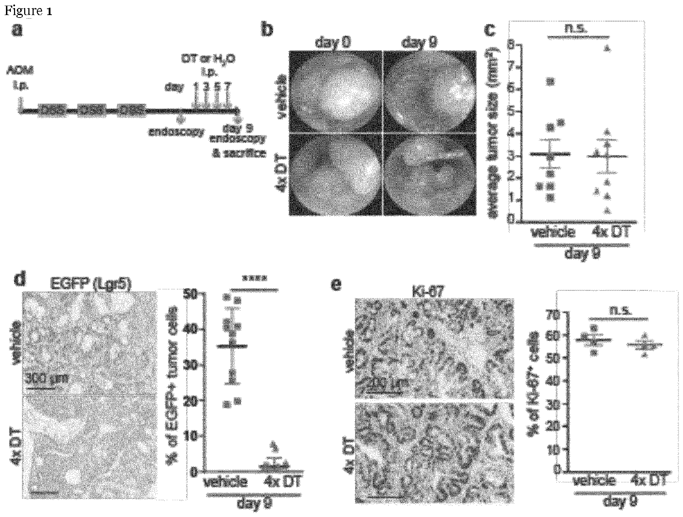

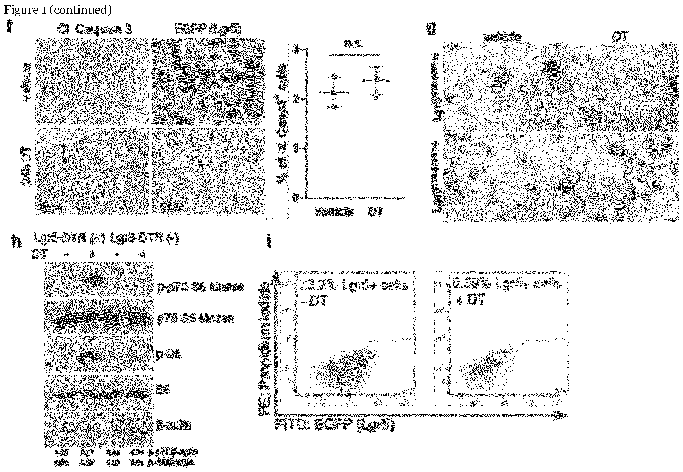

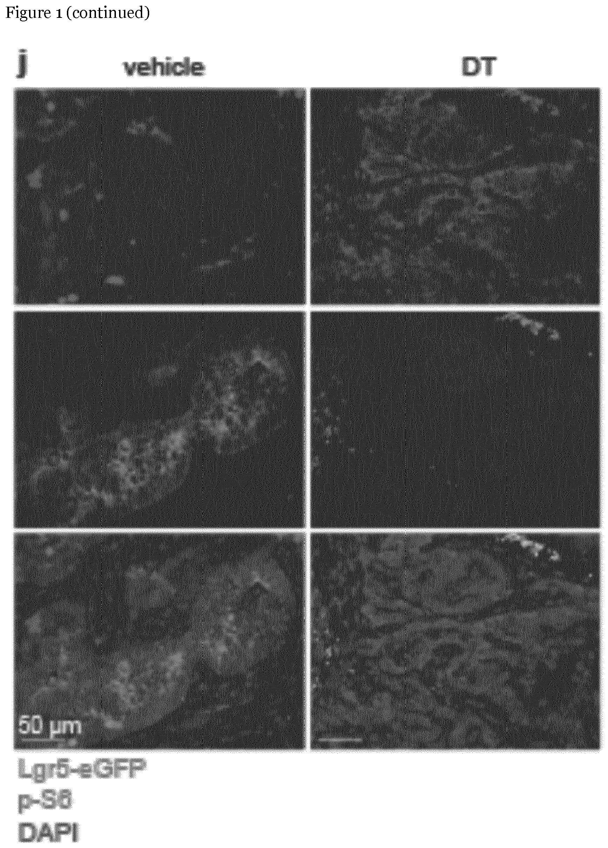

Figure 1 shows that the loss of Lgr5+ cells is compensated by mTORC1 activation in neighboring cells. a, Schematic representation of the AOM/DSS protocol for induction of colon tumors and time points of diphteria toxin (DT) administration to deplete Lgr5+ cells. b, Mini-endoscopy images of colon tumors at day 0 and on day 9 after 4 injections of either DT (50 µg/kg body weight) or vehicle (H2O) as control. Representative images are shown from at least three independent experiments. c, Average tumor size was counted on serial haematoxylin and eosin (H&E) stained sections on day 9 after 4 injections of either DT or vehicle. Data are mean ± SD, n ≥ 8/treatment. p>0.05 (n.s., not significant) by two-tailed Student's t-test. d, Immunohistochemical analysis and quantification of Lgr5-EGFP expression in GFP-stained colon tumor from Lgr5EGFP-DTR(+) mice on day 9 treated with DT as indicated in (a). Representative images are shown (n ≥ 8/treatment, scale bar = 300 µm). Data are mean ± SD, n ≥ 8/treatment. ****p<0.0001 by two-tailed Student's t-test. e, Immunohistochemical analysis of Ki-67 expression in colon tumors on day 9 after 4 injections of either DT or vehicle and quantification of Ki-67+ epithelial cells in tumors. Representative images are shown from four independent experiments (n = 4/treatment, scale bar = 200 µm). Data represent mean ± SD, n = 4/treatment. p>0.05 (n.s., not significant) by two-tailed Student's t-test. f, Immunohistochemical analysis of cleaved caspase 3 and GFP expression and quantification of cleaved caspase 3 positive cells in colon tumors 24h after injections of either DT or vehicle. Representative images are shown from three independent experiments (n = 3/treatment, scale bar = 200 µm). Data are mean ± SD, n = 3/treatment. p>0.05 (n.s., not significant) by two-tailed Student's t-test. g, AOM/DSS colon tumor organoids from Lgr5EGFP-DTR(-) and Lgr5EGFP-DTR(+) mice were treated with DT (20 ng/ml) for 24 h. Representative images are shown from two independent experiments (scale bar = 200 µm). h, Immunoblot analysis of Lgr5EGFP-DTR(+) and Lgr5EGFP-DTR(-) tumor organoids treated with DT for 24 h. Representative results are shown (n = 3). i, Flow cytometric analysis of Lgr5-EGFP+ tumor cells from Lgr5EGFP-DTR(+) tumor organoids 24h after DT treatment. Representative results are shown (n > 3). j, Immunofluorescence analysis of p-S6 (red) and GFP (Lgr5, green) expression in tumor tissues of vehicle or DT (50 µg/kg) treated AOM/DSS mice 24h after injection. Representative images are shown (n = 3, scale bar = 50 µm). k, Lgr5EGFP-DTR(+) tumor organoids were treated with DT (20 ng/ml), rapamycin (10 µM) or DT plus rapamycin for 24 h. Representative images are shown from three independent experiments. 1, Lgr5EGFP-DTR(+) tumor organoids were treated with DT (20 ng/ml), rapamycin (10 µM) or DT plus rapamycin for 24 h, re-seeded and cultured for 72 h. Percentage of reseeding capacity compared to vehicle treated organoids is indicated. Data are mean ± SEM, n = 4. *** p<0.001 by 1-way ANOVA with Bonferroni's multiple comparison test. m, Control and raptor knockdown Lgr5EGFP-DTR(+) tumor organoids were passaged and counted after 72 h. Percentage of organoid forming capacity compared to control organoids is indicated. Data from one representative experiment are shown and represent mean ± SD, n = 3. p>0.05 by two-tailed Student's t-test. n, Control and raptor knockdown Lgr5EGFP-DTR(+) tumor organoids were treated with DT (20 ng/ml) for 24 h and, re-seeded and cultured for 72 h. Percentage of reseeding capacity compared to control organoids is indicated. Data from one representative experiment are shown and represent mean ± SD, n = 3. *** p<0.001 by two-tailed Student's t-test. o, Efficiency of shRNA mediated knockdown of raptor in Lgr5EGFP-DTR(+) tumor organoids was determined by quantitative RT-PCR. Data are mean ± SD, n = 3. * p<0.05 by two-tailed Student's t-test.
Figure 2 shows that inhibition of mTOR induces tumor collapse upon Lgr5+ cell depletion. a, Schematic representation of AOM/DSSATKN tumor organoid generation. b, Immunoblot analysis of AOM/DSSATKN tumor organoids treated with rapamycin (10 µM) alone or in combination with DT (20 ng/ml) for 24 hours. Data from one representative experiment are shown (n =3). c, AOM/DSSATKN tumor organoids were injected subcutaneously and mice were treated with single dose of DT (50 µg/kg), rapamycin (10 mg/kg) or both in combination for 24 h once tumor reached 200 mm3. Representative images of H&E (n = 3, scale bar = 200 µm). d, AOM/DSSATKN tumor organoids were injected subcutaneously and mice were treated with single dose of DT (50 µg/kg), rapamycin (10 mg/kg) or both in combination for the indicated timepoints. Tumor tissues were subjected to immunofluorescence analysis of cleaved caspase 3 (red) and GFP (Lgr5, green) expression. Representative images from at least 2 mice/timepoint are shown, (scale bar = 100 µm). e, Treatment regimen of subcutaneous AOM/DSSATKN tumors. Tumor organoids were injected up to 15 days before DT and rapamycin were applied. DT was applied every two days, rapamycin was injected once per day. f, Tumor growth rate of subcutaneous AOM/DSSATKN tumors in response to treatment as indicated in (e). Mean tumor volumes ± SEM are shown, n = 5 for vehicle, n =5 for DT, n = 6 for rapamycin and n = 9 for rapamycin + DT. ** p<0.01 , *** p<0.001 by 2-way ANOVA with Bonferroni's multiple comparison test. g, Representative images of subcutaneous AOM/DSSATKN tumors on day 8 treated with DT, rapamycin or DT/rapamycin as indicated in (e), (scale bar = 5mm). h, Representative images of p-S6 (red)-stained small intestine sections from C57/B6 WT mice treated with single injection of AOM (10 mg/kg) or 12 grey irradiation and sacrificed 8 hours post treatment (n = 3). i, Representative images of p-S6 (red)-stained small intestine sections from C57/B6 WT mice treated with single injection of 5-FU (20mg/kg) or 5-FU in combination with rapamycin (10 mg/kg) sacrificed 24 hours post treatment (n = 3). j, Immunoblot analysis of AOM/DSSATKN tumor organoids treated with chemotherapeutic drug 5-FU (20 µM), rapamycin (10 µM) or 5-FU plus rapamycin for 24 hours. Representative results are shown (n = 3). k, AOM/DSSATKN tumor organoids were treated with chemotherapeutic drug 5-FU (20 µM), rapamycin (10 µM) or 5-FU plus rapamycin for 48 hours, re-seeded and cultured for 72 h. Percentage of reseeding capacity compared to vehicle treated organoids is indicated. Data are mean ± SD, n = 3. One representative of three experiments is shown. * p<0.05 , *** p<0.001 by 1-way ANOVA with Bonferroni's multiple comparison test. 1, Immunoblot analysis of human tumor organoids treated with 5-FU (20 µM), rapamycin (10 µM) or 5-FU plus rapamycin for 24 hours. Representative results are shown (n = 3). m, Human tumor organoids were treated with 5-FU (20 µM), rapamycin (10 µM) or 5-FU plus rapamycin for 48 hours and then re-seeded in ENR containing medium for 72 h. Percentage of reseeding capacity compared to vehicle treated organoids is indicated. Data are mean ± SD, n = 3. One representative of three experiments performed is shown. * p<0.05 , *** p<0.001 by 1-way ANOVA with Bonferroni's multiple comparison test. n, Treatment regimen of subcutaneous AOM/DSSATKN tumors. Tumor organoids were injected up to 15 days before 5-FU and rapamycin were applied. 5-FU (20 mg/kg) was applied 4 times every two days, rapamycin (10 mg/kg) was injected once per day. o, Tumor growth rate of subcutaneous AOM/DSSATKN tumors in response to treatment as indicated in (n). Mean tumor volumes ± SD are shown, n > 5 for each condition. * p<0.05 , *** p<0.001, **** p<0.0001 by 2-way ANOVA with Bonferroni's multiple comparison test. p, Representative images of subcutaneous AOM/DSSATKN tumors on day 8 treated with 5-FU, rapamycin or 5-FU/rapamycin as indicated in (e), (scale bar = 5mm).

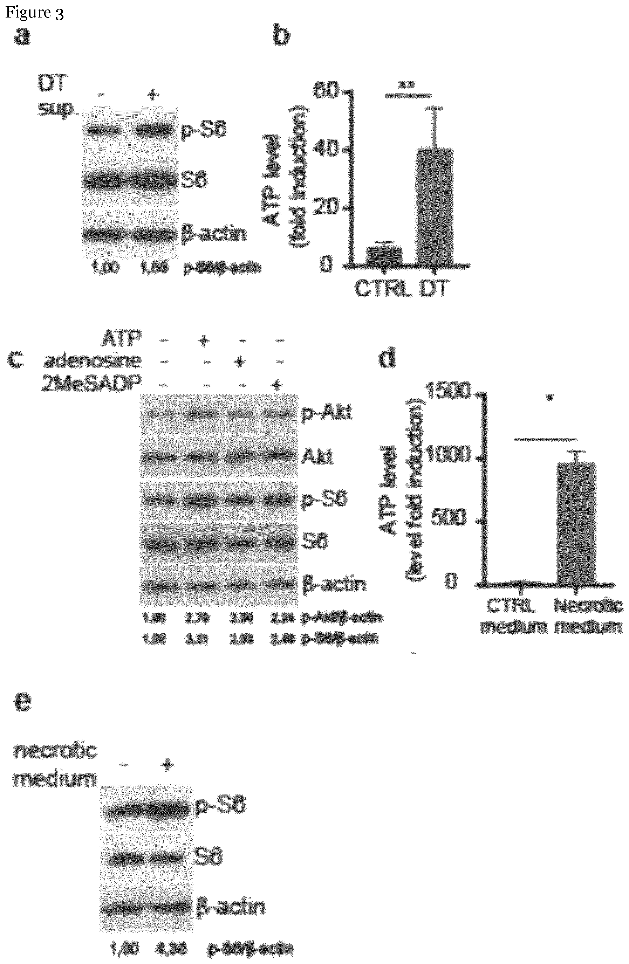

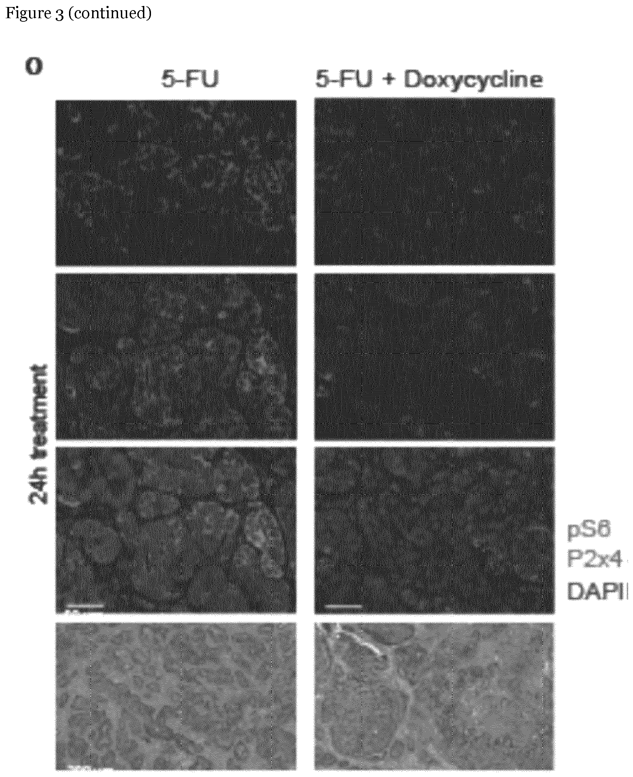

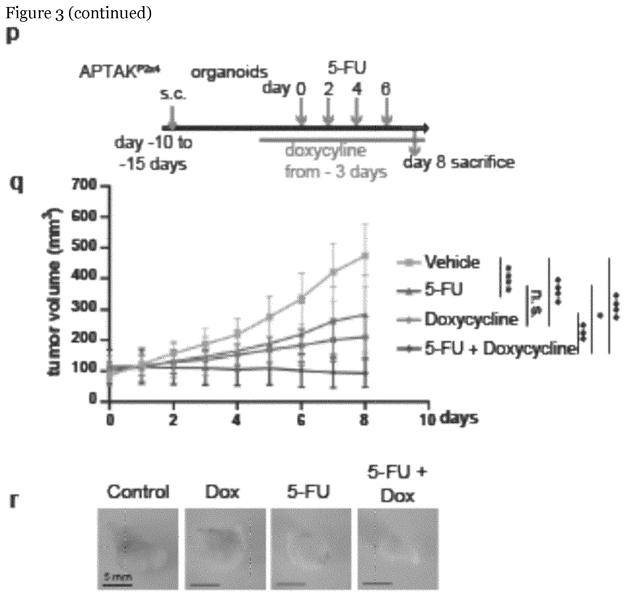

Figure 3 shows that S6 phosphorylation is triggered by ATP release from dying cells. a, Immunoblot analysis of Lgr5EGFP-DTR(-) tumor organoids treated for 24 hours with supernatant from vehicle- or DT-treated Lgr5EGFP-DTR(+) tumor organoids for 24 hours. Representative results are shown (n = 2). b, Analysis of ATP content in supernatants of vehicle or DT treated organoids 6h after treatment. Data are mean ± SD, n = 7. * p<0.05 by two-tailed Student's t-test. c, Immunoblot analysis of Lgr5EGFP-DTR(-) tumor organoids treated with ATP (100 µM), 2MeS ADP (100 µM) and adenosine (100 µM) for 30 min. Representative results are shown (n = 3). d, Analysis of ATP content in control medium or necrotic medium. Data are mean ± SD, n = 3. * p<0.05 by two-tailed Student's t-test. e, Immunoblot analysis of Lgr5EGFP-DTR(+) tumor organoids treated with supernatant of necrotic cells for 4 hours. Representative results are shown (n = 3). f, Immunoblot analysis of Lgr5EGFP-DTR(+) tumor organoids treated with DT for 6h in the presence or absence of Apyrase. Representative results are shown (n = 3). g, Immunoblot analysis of Lgr5EGFP-DTR(+) tumor organoids treated for 2h with necrotic medium in the presence or absence of Apyrase. Representative results are shown (n = 3). h, Lgr5EGFP-DTR(+) tumor organoids were treated with AZD5363 (10 µM), purinergic receptor inhibitors (50 µM theophylline; 20 ng/ml Pertussis toxin and 30 µM PPADS) and 5-FU (10 µM) as indicated for 48 h, re-seeded in and cultured for 72h. Percentage of reseeding capacity compared to vehicle treated organoids is indicated. Data are mean ± SD, n = 4 replicates. One representative of four experiment performed is shown.). *** p<0.001 by 1-way ANOVA with Bonferroni's multiple comparison test. i, Relative mRNA expression levels of the indicated genes in AOM/DSS colon tumors organoids determined by qRT-PCR (n=3). j, Immunoblot analysis of APTAK colon tumor organoids treated with ATP (100µM) in the presence or absence of 5-BDBD (10µM) for 15 min. Representative results are shown (n = 3). k, AOM/DSSATKN tumor organoids were treated with 5-BDBD at indicated concentrations and 5-FU (20 µM) for 48 h, re-seeded in and cultured for 72h. Percentage of reseeding capacity compared to vehicle treated organoids is indicated. Data are mean ± SD, n = 3 replicates. One representative of three experiment performed is shown. n.s. p>0,05, * p<0.05 ***, p<0.001, **** p<0.0001 by 1-way ANOVA with Bonferroni's multiple comparison test. 1, Immunoblot analysis of doxycycline inducible P2x4 knockdown APTAK colon tumor organoids treated with 5-FU (20 µM). Organoids were cultured in medium -/+ doxycycline prior to the experiment. Representative results are shown (n = 3). m, Doxycycline inducible P2x4 knockdown APTAK organoids were treated with 5-FU (20 µM) for 48 hours, re-seeded and cultured for 72 h. Organoids were cultured in medium -/+ doxycycline prior to the experiment. Percentage of reseeding capacity compared to vehicle treated organoids is indicated. Data are mean ± SD, n =3. One representative of three experiment performed is shown. n.s. p>0,05, * p<0.05 ***, p<0.001, **** p<0.0001 by 1-way ANOVA with Bonferroni's multiple comparison test. n, Efficiency of shRNA mediated knockdown of P2x4 in Lgr5EGFP-DTR(+) tumor organoids was determined by quantitative RT-PCR. Data are mean ± SD, n = 3. **** p<0.0001 by two-tailed Student's t-test. o, APTAKshP2x4 tumor organoids were injected subcutaneously and mice were treated with single dose of 5-FU in the presence or absence of doxycycline 24 h and tumor tissues were analyzed by immunofluorescence against p-S6 (red) and P2x4 (green). One representative images is shown (n = 3, scale bar = 50 µm). p, Treatment regimen of subcutaneous APTAKshP2x4 tumors. Tumor organoids were injected up to 15 days before 5-FU treatment, doxycycline treatment (0,5 mg/ml of drinking water) was started 3 days prior to the first 5-FU application. 5-FU (20mg/kg) was applied four times every two days. q, Tumor growth rate of subcutaneous APTAKshP2x4 tumors in response to treatment as indicated in (p). Mean tumor volumes ± SD are shown, n > 5 for each condition. n.s. >0.05, * p<0.05 , *** p<0.001, **** p<0.0001 by 2-way ANOVA with Bonferroni's multiple comparison test. r, Representative images of subcutaneous APTAKshP2x4 tumors tumors on day 8 treated with 5-FU in the presence or absence of doxycycline as indicated in (p), (scale bar = 5mm).
Figure 4 shows that paracrine activation of apoptosis induced by cell death and mTOR signaling blockade is counteracted by inhibition of ROS. a, AOM/DSSATKN tumor organoids were treated with chemotherapeutic drug 5-FU (20 µM), rapamycin (10 µM) or 5-FU plus rapamycin in the presence or absence of the apoptosis inhibitor zVAD (50µM) for 48 hours, re-seeded and cultured for 72 h. Percentage of reseeding capacity compared to vehicle treated organoids is indicated. Data are mean ± SD, n = 3. One representative of four experiments is shown. n.s.p>0.05, * p<0.05 , *** p<0.001 by 1-way ANOVA with Bonferroni's multiple comparison test. b, AOM/DSSATKN tumor organoids were treated with chemotherapeutic drug 5-FU (20 µM), rapamycin (10 µM) or 5-FU plus rapamycin in the presence or absence of NAC (2mM), anti-HMGB1 antibody (5µg/ml), Enbrel (10 mg/ml, C176/C178 (0,5 µM), Anakinra (100ng/ml) for 48 hours, re-seeded and cultured for 72 h. Percentage of reseeding capacity compared to vehicle treated organoids is indicated. Data are mean ± SD, n = 3. One representative of four experiments is shown. n.s.p>0.05, * p<0.05 , **** p<0.0001 by 1-way ANOVA with Bonferroni's multiple comparison test. c, AOM/DSSATKN tumor organoids were treated with chemotherapeutic drug 5-FU (20 µM), rapamycin (10 µM) or 5-FU plus rapamycin in the presence or absence of NAC (2mM), Trolox (ImM) or BHA (10 µM) for 48 hours, re-seeded and cultured for 72 h. Percentage of reseeding capacity compared to vehicle treated organoids is indicated. Data are mean ± SD, n = 3. One representative of four experiments is shown. n.s. p>0.05, * p<0.05 , **** p<0.0001 by 1-way ANOVA with Bonferroni's multiple comparison test. d, Fluorescence microscopic and FACS analysis of ROS production using DCFDA (green) in control and 5-FU (20µM, 20h) treated human colon organoids derived from healthy or tumor tissue. One representative of four experiments is shown (scale bar = 1mM). e, Immunoblot analysis of AOM/DSSATKN tumor organoids treated with rapamycin (10 µM) alone or in combination with DT (20 ng/ml) in the presence or absence of NAC (2mM) for 24 hours. Data from one representative experiment out of 3 independent experiments are shown. f, AOM/DSSATKN tumor organoids were injected subcutaneously and mice were treated with single dose of DT (50 µg/kg), rapamycin (10 mg/kg) or both in combination in the presence or absence of NAC (0,5% in drinking water) for 12h once tumor reached 200 mm3. Tumor tissues were then subjected to immunofluorescence analysis using antibodies against cleaved Caspase 3. One representative out of three independent experiments is shown (n = 3, scale bar = 200 µm). g, Treatment regimen of subcutaneous APTAKshP2x4 tumors. Tumor organoids were injected up to 15 days before 5-FU treatment, NAC treatment (0,5% in drinking water) was started 3 days prior to the first 5-FU application. 5-FU (20mg/kg) was applied four times every two days. h, Tumor growth rate of subcutaneous APTAKshP2x4 tumors in response to treatment as indicated in (g). Mean tumor volumes ± SD are shown, n > 5 for each condition. n.s. >0.05, *** p<0.001, **** p<0.0001 by 2-way ANOVA with Bonferroni's multiple comparison test. i, Representative images of subcutaneous APTAKshP2x4 tumors tumours on day 8 treated with 5-FU and rapamycin in the presence or absence of NAC as indicated in (p), (scale bar = 5mm). j, Representative images H&E stainings of subcutaneous APTAKshP2x4 tumors tumors on day 8 treated as described in (g) (scale bar = 200µm). k, Schematic representation of how dying cells activate mTOR survival pathway in adjacent cells in order to counteract apoptosis induction by increased levels of ROS and to maintain tumor integrity.

### EXAMPLES

### Methods

### Animal studies

All experiments involving animals were reviewed and approved by Regierungsprasidium Darmstadt, Darmstadt, Germany. Lgr5^{EGFP-DTR(+)} mice were described previously (3,4). .NOD.Cg-*Prkdc^{scid}Il2rg^{tm1Wjl}l*SzJ (NSG) mice were purchased from Jackson Laboratory and colony was maintained at animal facilities of Georg-Speyer-Haus, Frankfurt or MFD diagnostics, Wendelsheim. 8-10 weeks old male or female NSG mice were used for experiments. NOD/Shiscid, IL-2Rynull (NOG) mice (7-12 weeks of age, male) were obtained from the Central Institute for Experimental Animals (CIEA, Japan) and maintained at animal facilities of Keio University.

### AOM/DSS induced colon tumorigenesis and mini-endoscopy

Colon tumors were induced as previously described33. Mice (8-10 weeks old) were injected intraperitoneally (i.p.) with AOM (10 mg/kg; Sigma, #A5486) and after 5 days, 2% DSS (molecular weight, 36-50 kDA; MP Biochemicals, #SKU 0216011090) was given in the drinking water for 5 days, followed by 14 days for regular water. The DSS treatment was repeated for two additional cycles. Mini endoscopy was performed at around day 80 and after the treatments on anesthetized mice using a mouse mini endoscopy system (Karl Storz). Recorded videos from endoscopy system were opened and viewed in VLC Media Player and still images were captured from these videos.

### Xenograft experiments

Organoids were collected using ice cold Cell Recovery Solution (Corning, #354253) and mechanically dissociated into small clusters. Dissociated organoid cells were resuspended in PBS, admixed with 50% Matrigel (Corning, #356231) in a final volume of 100 µl, and injected subcutaneously in the right flank of NSG mice. Tumor size was monitored with a digital caliper and tumor volumes were calculated according to the formula (length × width × width)/2. At the end of experiments mice were humanely euthanized to collect tumors.

### Diphtheria toxin and other in vivo treatments

DT (Millipore, #322326) treatment was performed as previously described (3, 4). Briefly, DT was injected intraperitonially at a dose of 50 µg/kg for the time indicated in individual experiments. mTOR inhibitor rapamycin (LC Labs, #-5000) was dissolved in 5% Ethanol, 5% PEG400 and 5% Tween 80 in PBS and injected i.p. at 10 mg/kg once a day for indicated time. 0,5% N-acetylcysteine (Sigma-Aldrich, #A91165) was applied ad libitum in drinking water. 0,5 mg/ml Doxycycline (Sigma#D9891) was applied ad libitum in drinking water containing 3% Sucrose. 5-FU (Sigma#F6627) was dissolved in sterile H2O and injected intraperitoneally for the time indicated in individual experiments at a concentration of 20 mg/kg.

### Tumor organoid culture

Colon tumor organoids were cultured as previously described (27). AOM/DSS induced colon tumors were harvested by opening the colon longitudinally and dissociated enzymatically for 30-40 min in digestion media containing 1 mg/ml collagenase I (Sigma-Aldrich), 0.5 mg/ml Dispase (Roche), 50 µg/ml DNase (Sigma-Aldrich) in RPMI medium containing 2% FCS. Supernatant was washed and filtered through a 70 µM cell strainer. Cells were then embedded in Matrigel (Corning, #356231) and cultured in Advanced Dulbecco's modified Eagle's medium/F12 (Invitrogen, #12634-028) supplemented with Glutamax (Invitrogen, #35050038), Hepes (Invitrogen, #15630056), Pen/Strep (Invitrogen, #15140122), N2 supplement (Invitrogen, #17502048), B27supplement (Invitrogen, #17504044) and n-Acetylcysteine (Sigma-Aldrich, #A91165). EGF (Invitrogen, #PMG8045), RspoI and noggin were added as niche factor. To culture ATKN organoids, puromycin (2 µg/ml), Hygromycin (200 µg/ml) and blasticidin (5 µg/ml) was added to culture medium. APTAK tumor organoids were generated by introduction of Apc Δ/Δ; Trp53 Δ/Δ; Tgfbr2 Δ/Δ; myristoylated human Akt and KrasG12D in wildtype colon organoids (Heichler et al. STAT3 activation through IL-6/IL-11 in cancer-associated fibroblasts promotes colorectal tumour development and correlates with poor prognosis. Gut. 2020 Jul;69(7): 1269-1282. doi: 10.1136/gutjnl-2019-319200. Epub 2019 Nov 4. PMID: 31685519) and cultured in organoid medium containing puromycin (2 µg/ml), Hygromycin (200 µg/ml). LGR5-iCapspase9-T2A-tdTomato knock-in colon cancer organoids were established as previously described (21). LGR5-iCT organoids were cultured without RspoI.

### Organoids treatments and organoid harvesting

*In vitro* Lgr5+ cells ablation experiments, organoids were treated with 20 ng/ml DT (Millipore, #322326) in the culture medium. For human LGR5+ cells ablation, we treated LGR5-iCT organoids with 500 nM BB homodimerizer. Other treatments were performed as described in each figure legends. Rapamycin (10 µM, Selleckchem, #S1039), PH797804 (2 µM, Tocris, #5866), SP600125 (5 µM, Sigma, #S5567), 5-BDBD (1µM and 10µM, Tocris#3579) NF279 (10 µM, Tocris, #1199), PPADS (60 µM, Santacruz, #sc-202770), PTx (40 ng/ml, Tocris, #3097), NAC (2mM, Sigma-Aldrich, #A91165), Trolox (1mM, Tocris#6002), BHA (10 µM, Sigma#B1253), theophyllin (50 µM, Sigma, #T1633), A438079 (10 µM, Sigma, #A9736),, 2'3' cGAMP (10 and 30 µg/ml, Invivogen, #tlrl-nacga23), ATP (100 µM, Sigma, #A6419), 2MeS-ADP (100 µM, Tocris, #1624), adenosine (100 µM, Sigma, #A92251), , 5-Fluorouracil (20 µM, Sigma, #F6627-5G); C-176 and C-178 (500 nM, Ablasser Lab18), Doxycycline (Sigma#D9891), anti-HMGB1 antibody (5µg/ml), Enbrel (Etanercept, 10 mg/ml), Anakinra (Kineret, 100ng/ml). After the treatment, organoids were collected in ice-cold Cell Recovery Solution (Corning, #354253) and pelleted by centrifugation. To prepare protein lysates for western blot analysis, organoid pellet was washed twice with cold PBS and pellet was snap frozen or lysed in lysis buffer.

### Measurement of ROS and ATP level

ATP level in organoid supernatant or necrotic medium was measured using the CellTiter-Glo 2.0 Cell Viability Assay (Promega#G9242) according to the manufacturers instruction. ROS levels were measured using H2DCFDA (ThermoFisher #D339). Briefly, organoids were treated as indicated with 5-FU, stained with H2DCFDA and then analyzed for H2DCFDA positivity using fluorescence microscopy or FACS.

### Necroptotic supernatant transfer experiment

Murine colorectal cancer cells (CMT93 and CT26) and human colorectal cancer cells (HCT116 and RKO) were cultured overnight in DMEM medium containing 2% FCS and culture supernatant was collected with or without cells and snap frozen in liquid nitrogen for 5 minutes, thaw at 37°C and filtered with 0.45 µM filters. Filtered control supernatant (without cells) and necroptotic supernatant (filtered supernatant of culture medium collected with cells) was transferred to 60-70% confluent cells (cultured overnight in DMEM medium containing 2% FCS). Cells were collected in lysis buffer at 2 h and 4 h time points for western blot analysis. For necroptotic supernatant transfer experiment in organoids control and necroptotic supernatant was prepared from CT26 cells cultured in Advanced DMEM/F12 without serum overnight and transferred to Lgr5^{EGFP-DTR(+)} tumor organoids. Organoids were collected after 4 h and lysed in lysis buffer for Western blot analysis.

### Western blotting

Collected organoids were lysed in the lysis buffer described elsewhere33. Protein content was quantified using protein assay dye reagent (Bio-Rad, #5000006) and 40 µg of total protein lysates in Laemmli buffer (Bio-Rad, #161-0747) were separated on SDS-PAGE, transferred to 0.45 µm PVDF membrane (Millipore, #IPVH00010) and blocked with 5% non-fat milk and 1% BSA in PBS at RT for 1 h. After blocking membranes were incubated at 4oC overnight with primary antibodies. The following antibodies were used: cleaved caspase 3(#9661), cleaved PARP (#9548), phosphor-histone H2a.X (#2577), phospho-p70 S6 kinase (#9205), p70 S6 kinase (#9202), phospho-S6 (#2211), S6 (#2217), phospho-p38 (#9211), p38 (#9112), phospho-ERK (#4370), ERK (#9102), phospho-cJUN (#9261), cJUN (#9165), c-Myc (#5605), cyclin-D1 (#2922) from Cell Signaling, P2x4 (Abcam#134559) and Sting (Abcam, #ab92605). Actin (Sigma, #A4700) was used as loading control. After washing with PBS, membranes were incubated with appropriate HRP-conjugated secondary antibodies for 1h at RT and membranes were developed using SuperSignal West Pico Chemoluminescence Substrate (Thermo Fisher Scientific, #34580).

### FACS

For the FACS analysis, organoids were collected in ice-cold Cell Recovery Solution, washed with cold PBS and dissociated to single cells using Accutase (Sigma, #A6964) at RT for 5 min, filtered through a 40 µM cell strainer and resuspended in FACS buffer (1 mM EDTA and 1% FCS in cold PBS). Propidium Iodide (Thermo Fisher, #BMS500PI) was added to single cell suspension for exclusion of non-viable cells in flow cytometry. Samples were acquired using BD LSRFortessa^{™} cell analyzer (BD Bioscience) and analyzed using FlowJo v9 (FlowJO LLC). For colon fibroblasts, DSS-colon fibroblasts and colon tumor fibroblasts, tissue was harvested, washed in PBS containing Pen/Strep antibiotics, minced using sterile scalpels and digested in digestion medium as described above. Supernatant was washed and filtered through a 40 µM cell strainer. Cells were stained using following antibodies: CD45 APC (#17-0451), CD31 eFlour450 (#48-0311), EpCAM PE-Cy7 (#25-5791-80), PDGFRα PE (#12-1401-81) from eBiosciensce. Viability dye eFluor 780 (eBiosciensce #65-0865-14) was included for the exclusion of non-viable cells. Samples were acquired and sorted using BD FACSAriaTM Fusion sorter. For RNA extraction, cells were directly sorted in the RLT lysis buffer and RNA was isolated using RNeasy Micro Kit (Qiagen, # 74004).

### siRNA mediated knockdown

60-70% confluent CT26 cells were transfected with 25 nM siRNA smartpool against mouse Rheb (#L-057044-00), RagA(#L-057667-01) or RagB (#L-066440-01) siRNA(Dharmacon) using DharmaFECT (Dharmacon) and Lipofectamine 2000 (Invitrogen) transfection reagents. RNA was purified after 48 h later to confirm the knockdown efficiency. For supernatant transfer experiments, culture medium was changed after 16 h of transfection, and supernatant was collected 72 h later, filtered and stored at 4°C.

### RNA extraction and qRT-PCR

For RNA extraction organoids were harvested directly in RLT buffer, RNA extraction was performed using RNeasy Mini kit (Qiagen, #74106) according to the manufacturer's instructions. Total RNA (1-2 µg) was reverse transcribed into cDNA using a Super script II reverse transcriptase (Invitrogen, #18064-071) and OligodT (Invitrogen, #18418020). Real-Time quantitative PCR was performed using FastStart Universal SYBR Master Mix (Roche, # 4913914001) in 20 µl total volume on a StepOne Plus Real-Time PCR system (Applied Biosystems). Relative gene expression levels were quantified by using cyclophilin as a housekeeping gene (2[Ct cyclophilin - Ct target gene]).

### Immunostaining and in situ hybridization

Immunostaining was performed as previously described (35). Briefly, tissues were fixed with 4% paraformaldehyde. Six µm OCT frozen tissue sections or 5 µm paraffin-embedded tissue sections were processed for immunostaing using a standard histological protocol. Following primary antibodies were used: EGFP (Abcam, #ab6673), tdTomato (Rockland, #600-401-379), cleaved caspase 3 (Cell Signaling, #9579), Ki67 (Leica Bioststems, #NCL-L-Ki67-MM1), phospho-S6 (Cell Signaling, #2211),P2x4 (Abcam#134559). Sections were then incubated with HRPconjugated antibodies (Dako) and signal was detected with DAB reaction. For immunofluorescence staining after primary antibody incubation, sections were incubated with flourochrome-labelled secondary antibodies (Invitrogen) and sections were mounted with ProLong Gold Antifade Mountant with DAPI (Invitrogen, #P36931). Images were captured on a Zeiss microscope.

### Plasmids, Lentivirus and retrovirus production

Top two P2x4 and Raptor shRNA sequences were obtained from and cloned into mir-E based retroviral backbone as previously described (36). K-ras G12D was cloned in pBABE-puro (Addgene plasmid #1764). An IRIS-AKT (myristoylated) was cloned downstream of hygromycin cassette in pMSCV-rtTA3-PGK-Hygro (kindly provided by Lars Zender, Tubingen). Retroviral particles were produced by co-transfection of retroviral plasmids with packaging plasmid pCL-ECO (Addgene plasmid #12371) to HEK293T cells using the calcium phosphate method. Notch intracellular domain (NICD) was cloned in LentiCas9-BSD (Addgene plasmid #52962) by replacing Cas9. Lentiviral supernatants were prepared by co-transfection of Lenti-NICD-BSD plasmid, the packaging vector psPAX2 (Addgene plasmid #12260) and the envelope vector pMD2.G (Addgene, #12259) using Lipofectamine 2000. The culture medium was replaced 12 h after transfection and viral supernatant was collected 48 h later, filtered through a 0.45 µm PVDF filter (Millipore). Viral supernatants were concentrated by overnight centrifugation at 12,000 rpm at 4°C and viral pellets were resuspended in organoid culture medium. Single cells from organoids were infected with polybrene-supplemented viral supernatant (8 µg/ml polybrene, Sigma). Cells were embedded in matrigel 4 h post infection and cultured in organoid culture medium supplemented with 10 µM Y-27632 (Sigma). 48 h post infection organoids were selected in 2 µg/ml puromycin, 200 µg/ml Hygromycin or 5 µg/ml Blasticidin for 5-7 days. Tgfr2 gRNA was cloned into gRNA cloning vector (Addgene plasmid #41824) as previously described (37). Single cells from organoids were co-transfected with gRNA plasmid and Cas9 expressing plasmid (Addgene plasmid #41815) using Lipofectamine 2000 as previously described (38).Tgfbr2 mutant organoids were selected in the absence of Noggin and presence of 50 ng/ml TGF-β (R&D Systems, # 7666-MB-005).

### Statistical analysis

No statistical method was used to predetermine the sample size for *in vivo* experiments. For subcutaneous transplantation experiments, mice were distributed among treatment groups when tumor size reached a volume of approximately 100-200 mm³. Statistical significance between two groups was determined by two-tailed Student's *t*-test and for more than two groups 1-way ANOVA or 2-way ANOVA with Bonferroni's multiple comparison test was performed (GraphPad Prism 4.03, GraphPad Software, Inc.). All the data in the graphs are shown as mean ± SEM, unless stated otherwise.

### Results

In the experiments as conducted in the context of the present invention, the inventors used AOM/DSS to induce colonic tumors in Lgr5^{EGFP-DTR(+)} mice (4). In contrast to subcutaneous tumors where loss of Lgr5+ cells leads to tumor stasis (3), the inventors could not detect a significant difference in tumor size or a decrease in tumor cell proliferation when Lgr5+ cells were depleted over a period of 9 days in this orthotopic setting (Fig. 1a-e). Also, the inventors could not see any change in the number of apoptotic cells in the tumors 24h after Lgr5 cell depletion (Fig. If).

Similarly, Lgr5+ cell deletion in CRC organoids derived from AOM/DSS treated Lgr5^{EGFP-DTR(+)} mice did not affect the gross morphology or growth rates of tumor organoids (Fig. 1g). These results strongly indicate that inflammation associated colonic tumors are not dependent on Lgr5+ cells. Notwithstanding the controversial role of Lgr5+ cells as cancer stem cells, it is surprising that colonic tumors are apparently not affected by this rapid loss of Lgr5+ cells which after all make up in average around 30% of tumor cells in the inventors' CRC model (Fig. 1d). Instead, acute depletion of Lgr5+ cells resulted in a transient increase of Ki-67+ tumor cells on day 4 (that is 24 hours after the second diphteria toxin (DT) application)

The inventors concluded that an activation of survival programs in Lgr5-negative cells helps the tumor to tolerate Lgr5 cell loss and to maintain tumor integrity. In order to test this finding and to characterize the molecular mechanism responsible for the survival of Lgr5- cells, the inventors screened organoids from AOM/DSS-induced colon tumors of Lgr5^{EGFP-DTR(-)} 24h after Lgr5+ cell depletion for the activation of known survival pathways. The inventors' immunoblot analysis indicated enhanced phosphorylation of c-Jun, p38, p70 S6 kinase as well as S6 ribosomal protein upon a single DT administration (Fig. 1 h). Phosphorylation of c-Jun, p38 and S6 could also be observed *in vivo* 24 hours after the first DT administration together with an increase of c-Myc and Cyclin D1 (Fig. 1j).

Notably, also in human cancer organoids Lgr5+ cell depletion (21) triggered S6 phosphorylation. To functionally confirm that the engaged MAP kinases or mTOR activation were indeed responsible for survival of Lgr5+ cell-depleted organoids the inventors applied SP600125, PH797804, or rapamycin to inhibit JNK, p38, and mTOR, respectively, in the presence or absence of DT. While the two MAPK inhibitors did not affect tumor organoid survival combined application of DT and rapamycin led to substantial cell death within 24 hours selectively in DTR expressing tumor organoids (Fig. 1k). Moreover, re-seeding of DT/rapamycin co-treated organoids resulted in a markedly reduced capacity to form organoids, which was not observed when either compound was applied individually (Fig. 1l).

In agreement with a critical role of mTORC1 in survival upon Lgr5+ cell loss, shRNA-mediated knockdown of raptor greatly diminished reseeding capacity of DT-treated tumor organoids, although raptor expression was reduced only by about 40% (Fig. 1m-o). In order to make sure that mTOR was activated in Lgr5- and not in remaining Lgr5+ cells the inventors confirmed an almost 100% deletion of Lgr5+ cells in tumor organoids 24h after DT treatment by FACS (Fig. 1i). Furthermore, the inventors performed a double staining of Lgr5-EGFP and pS6 in AOM/DSS tumor tissues which confirmed efficient Lgr5+ cell deletion and S6 phosphorylation exclusively in Lgr5-cells 24h post injection of DT (Fig. 1j). Downstream of mTOR the inventors identified c-myc and cyclin D1 expression to be markedly reduced when DT/rapamycin were applied, which was also associated with a marked reduction in tumor cell proliferation in AOM/DSS induced tumors.

Considering that the AOM/DSS model leads to the formation of benign adenomas only, the inventors intended to confirm that mTOR activation would also be relevant for tumor maintenance upon Lgr5+ cell-depletion in more advanced stages of colon cancer. Therefore, the inventors introduced either retrovirally or by lentiviral transduction a constitutively active form of AKT (myristoylated AKT), mutant K-Ras (K-rasG12D) as well as an active form of Notch1 (NICD) into AOM/DSS-induced tumor organoids from Lgr5^{EGFP-DTR(+)} mice. Moreover, *Tgfbr2* was knocked out by CRISPR/Cas9 mediated gene editing resulting in AOM/DSSATKN tumor organoids (Fig. 2a). Indeed, also in AOM/DSSATKN tumor organoids depletion of Lgr5+ cells led to S6 ribosomal protein phosphorylation that could be prevented by rapamycin (Fig. 2b) and combined DT/rapamycin administration led to loss of proliferating tumor cells in subcutaneously transplanted tumors within 24h. Notably, histological assessment of the tumor tissues indicated the absence of viable epithelial tumor cells in the tumor tissues of DT/rapamycin mice only 24h hours after treatment (Fig. 2c).

Rapidly proliferating cells require an increase in nucleotide supply, which is achieved by *de novo* synthesis of nucleotides that are incorporated into RNA and in proliferating cells into DNA. Interestingly, both Myc and mTOR signaling can stimulate *de novo* synthesis of nucleotides (5, 6). Inhibitors of inosine monophosphate dehydrogenase (IMPDH), a rate limiting enzyme in purine synthesis, has been used to target Myc- and mTORC1-dependent cancers (7, 8).

Given the activation of both mTOR and Myc signaling in Lgr5+ cell depleted tumors, the inventors explored whether an IMPDH inhibitor would mimic the effects of mTOR inhibition. Treatment of Lgr5^{EGFPDTR(+)} tumor organoids and s.c. transplanted AOM/DSSATKN tumors with the IMPDH inhibitor mizoribine in combination with DT-mediated depletion of Lgr5+ cells resulted in profound organoid collapse *ex vivo* and markedly reduced viable proliferative of AOM/DSSATKN tumors without affecting S6-ribosomal protein phosphorylation. These data suggested that *de novo* purine synthesis was required downstream of mTOR activation for maintaining tumor integrity in the absence of Lgr5+ cells.

As activation of mTOR signaling is known to suppress apoptosis (Hui L, Abbas T, Pielak RM, Joseph T, Bargonetti J, Foster DA. Phospholipase D elevates the level of MDM2 and suppresses DNA damage-induced increases in p53. Mol Cell Biol. 2004 Jul;24(13):5677-86. doi: 10.1128/MCB.24.13.5677-5686.2004. PMID: 15199126; PMCID: PMC480910), the inventors tested whether inhibition of the mTOR survival pathway upon killing of Lgr5+ triggered apoptosis in the remaining Lgr5- cells, responsible for the rapid and massive death of the remaining tumor cells. To this aim, the inventors assessed the kinetics of tumor cell apoptosis upon combination of Lgr5+ cell depletion and mTOR inhibition by immunostaining against cleaved caspase 3 on subcutaneously transplanted tumor tissue of mice treated for 6, 12 or 18h with DT in combination with rapamycin. Similar as in DT treated mice, in DT/rapamycin treated mice the inventors could detect only few Lgr5+ and Lgr5- apoptotic tumor cells 6h after treatment, a timepoint when Lgr5+ cells were still present in the tumor. However, 12h after treatment when Lgr5+ cells were sufficiently ablated from the tumor, the inventors detected massive apoptosis in the tumor tissue of DT/rapamycin treated animals whereas almost no apoptosis was detectable in animals treated only with DT (Fig. 2d).

Already, 18h post DT/rapamycin treatment, apoptosis was completed and most of the remaining Lgr5- tumor cells dead, as indicated by the lack of cleaved caspase 3 and the histology of the tumor tissues (Fig. 2d), suggesting that the Lgr5+ cell death induced mTOR signaling activation is critical for the survival of Lgr5- cells. Western blot analysis of colon tumor organoids using antibodies against p-γH2AX demonstrated increased DNA damage upon DT/rapamycin treatment and antibodies against cleaved caspase 3 and cleaved PARP confirmed increased apoptosis caused by the combination of Lgr5+ ablation and mTOR inhibition. As a consequence, prolonged rapamycin treatment plus Lgr5+ cell ablation caused marked tumor shrinkage of established tumors in contrast to single rapamycin or DT administration that individually applied led only to tumor stasis (Fig. 2e-g), confirming that the requirement of mTOR activation for tumor maintenance was independent of the underlying mutational load in tumors.

The observed paracrine mTOR activation may not represent a specific response to tumor stem cell death only. In line with this notion, the inventors confirmed strong S6 ribosomal protein phosphorylation in intestinal epithelial cells (IEC) when the inventors depleted Lgr5+ cells in unchallenged Lgr5^{EGFP-DTR(+)} mice without tumors, and the inventors also observed enhanced S6 phosphorylation in IEC when the inventors induced IEC apoptosis in a non-stem cell restricted manner using the carcinogen azoxymethane or a single dose of whole body irradiation (12 Gy) in untransformed wildtype mice (Fig. 2h). Based on this, the inventors concluded that combined mTOR inhibition and cell death induction could be employed therapeutically to enhance cytotoxic therapy response in tumors. Expectedly, 5-FU induced S6 phosphorylation in AOM/DSS tumor tissues and tumor organoids which was suppressed by administration of Rapamycin (Fig. 2i, j) and combination of rapamycin with 5-FU significantly suppressed reseeding capacity of tumor organoids (Fig. 2k, m).

Importantly, 5-rapamycin co-treatment suppressed mTOR activation and organoid survival also in human tumor organoids (Fig. 2l, m). Similar to Lgr5+ cell depletion, 5-FU treatment in combination with rapamycin significantly reduced tumor growth of subcutaneously transplanted tumors when compared to the control or each treatment alone (Fig. 2n-p).

Although it has already been shown that Lgr5 expression confers resistance to 5-FU based chemotherapy (Hsu HC, Liu YS, Tseng KC, Hsu CL, Liang Y, Yang TS, Chen JS, Tang RP, Chen SJ, Chen HC. Overexpression of Lgr5 correlates with resistance to 5-FU-based chemotherapy in colorectal cancer. Int J Colorectal Dis. 2013 Nov;28(11):1535-46. doi: 10.1007/s00384-013-1721-x. Epub 2013 Jun 20), the inventors aimed to confirm that mTOR activation by 5-FU treatment is independent of Lgr5+ cell death. The presence of Lgr5+ cells in the inventors' AOM/DSS tumor organoids is strongly dependent on the inhibition of BMP signaling and withdrawal of the BMP inhibitor Noggin from the inventors' standard organoid medium reduces the percentage of Lgr5+ below 1%. However, regardless of the absence of Lgr5+ cells, 5-FU treatment was able to induce phosphorylation of S6 in AOM/DSS turn or organoids, showing that mTOR activation does not require tumor stem cell death. The inventors furthermore wanted prove that the activation of mTOR signaling by cell death does not require a preceding inflammatory component. To this aim the inventors made use of genetically engineered APTAK tumor organoids, generated by introduction of Apc Δ/Δ; Trp53 Δ/Δ; Tgfbr2 Δ/Δ; myristoylated human Akt and KrasG12D in wildtype colon organoids (Heichler et al. STAT3 activation through IL-6/IL-11 in cancer-associated fibroblasts promotes colorectal tumour development and correlates with poor prognosis. Gut. 2020 Jul;69(7): 1269-1282. doi: 10.1136/gutjnl-2019-319200. Epub 2019 Nov 4. PMID: 31685519). Similar to the previous results in AOM/DSS tumor organoids, also here 5-FU led to mTOR dependent S6 phosphorylation and inhibition of mTOR signaling upon 5-FU treatment significantly reduced organoid reseeding capacity compared to 5-FU treatment alone.

Next, the inventors aimed to evaluate how Lgr5+ cell depletion could induce mTOR activation in neighboring Lgr5- cells. Cell death leads to the release of danger-associated molecular patterns (DAMPs) including various growth factors, inflammatory mediators, dsDNA and metabolites (9-11) that trigger inflammation and activate innate immunity to stop the damage and to orchestrate tissue repair and wound healing (12). To test whether DT-mediated killing of Lgr5+ cells leads to the release of DAMPs that could be responsible for paracrine mTOR activation, the inventors treated Lgr5^{EGFP-DTR(-)} tumor organoids for 22 h with supernatants from either vehicle- or DT-treated Lgr5^{EGFP-DTR(+)} tumor organoids and confirmed increased phosphorylation of S6 ribosomal protein in organoids exposed to supernatants from DT treated cells (Fig. 3a). Considering that DT induces apoptosis rather than other forms of cell death, the inventors hypothesized that release of either dsDNA or ATP could trigger S6 ribosomal protein phosphorylation. Because the inventors had observed an upregulated interferon response upon killing of Lgr5+ cells in AOM/DSS induced tumors (Extended Data Fig. 3a) similarly to s.c. transplanted tumors3 and which could also be observed in DT-treated tumor organoids, the inventors first focused on dsDNA that is known to initiate transcription of type-I interferons (IFNs) and interferon-stimulatory genes (ISGs) by activating the cGAS-STING pathway (13) which had recently also been shown to confer pro-tumorigenic and pro-metastatic properties when activated in tumor cells (14-17). Indeed, exogenous cGAMP induced phosphorylation of S6-ribosomal protein in a dose dependent manner. However, neither selective STING inhibitors C-176 and C-17818 nor CRISPR-mediated knockout of *Sting* in Lgr5^{EGFP-DTR(+)} AOM/DSS-induced tumor organoids was able to prevent S6-ribosomal protein phosphorylation after Lgr5+ cell depletion, ruling out an essential contribution of dsDNA to mTOR activation. Another DAMP released by dying cells is ATP, which the inventors could detect at elevated in the supernatant of DT treated organoids (Fig. 3b). Extracellular ATP can be hydrolyzed by ATP-hydrolyzing enzymes present in tumor microenvironment to generate ADP and adenosine that act as natural ligands for purinergic receptors (P2R) and adenosine receptors (P1R) and addition of either ATP or its hydrolyzed products induced phosphorylation of S6-ribosomal protein in Lgr5^{EGFP-DTR(-)} tumors organoids (Fig. 3c).

Importantly, also exposure of both murine and human colorectal tumor cells to supernatants of necrotic cells that were generated by freeze thaw cycles contained ATP and triggered a marked S6 phosphorylation (Fig. 3d) underscoring the notion that paracrine mTOR activation occurs independently of the type of cell death. Similarly, incubation of 3D cultured Lgr5^{EGFP-DTR(+)} tumor organoids with necrotic medium also resulted in S6 phosphorylation (Fig. 3e).

In confirmation of an important function of ATP for the paracrine activation of mTOR signaling by dying by Lgr5+ cells, the inventors were able to reduce the levels of S6 phosphorylation induced by DT treatment or necrotic supernatant by hydrolyzing the released ATP to AMP and inorganic phosphate using Apyrase (Fig. 3f,g). Mechanistically, mTOR activation by ATP was rather dependent on Rheb than on Rag GTPases as shown by a reduction of ATP induced S6 phosphorylation when Rheb was downregulated by siRNA in CRC cells. Downregulation of Rag A and B had no overt effect on p-S6 levels.

ATP signals via purinergic receptors of the P2 family, which comprises seven subtypes of P2X receptors and 8 subtypes of P2Y receptors (20). Blocking these receptors using a combination of nonselective P2 receptor antagonists in combination with 5-FU phenocopied the effect of the 5-FU/rapamycin double treatment on organoid survival (Fig. 3h). Furthermore, the non-selective P2 receptor antagonists PPADS and (PTx) blocked Lgr5+ cell death induced S6 phosphorylation. The inventors performed sequencing on tumor cells from DT treated Lgr5EGFP-DTR(-) 24h after DT treatment and compared the expression of P2 receptors to cells obtained from vehicle treated mice and found no overt difference in the expression of P2x, P2y receptors or connexin and pannexin hemichannels in DT treated animals. Of note, according to the inventors' sequencing data and confirmed by qPCR, P2x4 was remarkably higher expressed in murine tumor cells compared to all other P2 receptors (Fig. 3i). Also in human tumor organoids the inventors could detect much higher expression of P2x4 compared to other P2 receptors, however organoids from healthy human tissue showed similarly high expression levels of P2x4, indicating that P2x4 is in general the most prominent P2 receptor in IECs. Immunohistochemistry against P2x4 on mouse intestinal tissues confirmed strong and equal expression of P2x4 in normal and tumorigenic colon tissues.

Considering these expression data, the inventors tested whether blocking of this single P2 receptor might be enough to suppress ATP induced activation of the mTOR survival pathway. Indeed, chemical inhibition via the selective P2x4 inhibitor 5BDBD reduced phosphorylation of S6 by ATP in AOM/DSS tumor organoids (Fig. 3j), whereas using inhibitors against P2x1 (NF279) or P2x7 (A-438079) did not decrease ATP dependent S6 phosphorylation (Extended Data Fig. 3m). Accordingly, chemical inhibition of P2x4 in combination with 5-FU treatment led to decreased organoid survival in AOM/DSS and APTAK tumor organoids when compared to each treatment alone (Fig. 3k). Similarly, genetic knockdown of P2x4 in APTAK tumor organoids reduced mTOR activation induced by 5-FU and significantly reduced organoid survival when compared to P2x4 proficient organoids (Fig. 3l-n). *In vivo* the knockdown of P2x4 in subcutaneously transplanted tumors inhibited S6 phosphorylation by 5-FU treatment resulting in tumor cell death and significantly reduced tumor growth compared to P2x4 proficient tumors (Fig. 3o-r).

Altogether, the inventors' data show that ATP released by dying tumor cells trigger mTOR activation in adjacent tumor cells critical for their survival. However, in addition these cells need to receive a signal that triggers apoptotic cell death, which needs to be counteracted by mTOR. In line with this assumption, inhibition of apoptosis using zVAD (carbobenzoxy-valyl-alanyl-aspartyl-[O-methyl]-fluoromethylketone) rescued the negative effect of combined 5-FU/rapamycin treatment on organoid survival (Fig. 4a).

Due to the possibility to rescue organoid death by inhibition of apoptosis the inventors aimed to address which upstream signal induces apoptosis and tumor organoid death upon 5-FU/rapamycin treatment. To this aim the inventors screened inhibitors against potential proapoptotic DAMPs known to be released upon cell death such as N-acetylcysteine (NAC) against reactive oxygen species (ROS), anti-HMGB1 antibodies, the TNF-signaling inhibitor Enbrel, the STING inhibitors C176/C178 and the IL-1 signaling inhibitor Anakinra. Notably, only inhibition of ROS signaling by NAC was able to rescue the negative effect of 5-FU/rapamycin on organoid reseeding capacity (Fig. 4b), suggesting that ROS might be responsible for the increased DNA damage and apoptosis seen after chemotherapy when combined with inhibition of mTOR signaling. The identical effect of two other ROS inhibitors (the vitamin E analogue 6-hydroxy-2,5,7,8-tetramethylchroman-2-carboxylic acid (Trolox) and butylated hydroxytoluene (BHA)) on organoid survival additionally indicated ROS as critical mediator of organoid death in the inventors' scenario (Fig. 4c). By using the fluorescent dye 2',7'-dichlorofluorescin diacetate (DCFDA) to measure ROS levels in organoids the inventors were able to detect a tumor organoid specific ROS increase upon 5-FU treatment (Fig. 4d) able to induce DNA damage and apoptosis in remaining tumor cells.

Accordingly, the inventors' results demonstrating decreased DNA damage and apoptosis in tumor organoids treated with NAC in addition to DT and rapamycin when compared to DT/rapamycin treatment alone confirmed ROS as paracrine trigger of apoptosis (Fig. 4e) and also *in vivo* NAC reduced apoptosis in tumor tissues of DT/rapamycin treated animals (Fig. 4f). Most importantly and underscoring the role of ROS as mediator of tumor cell death the inventors were able to revert tumor regression in 5-FU/rapamycin treated mice by additional application of NAC (Fig. 4g-j).

### References

1. Barker, N., van Es, J. H., Kuipers, J., Kujala, P., van den Born, M., Cozijnsen, M. et al. Identification of stem cells in small intestine and colon by marker gene Lgr5. Nature 449, 1003-1007, (2007).
2. Schepers, A. G., Snippert, H. J., Stange, D. E., van den Born, M., van Es, J. H., van de Wetering, M. et al. Lineage tracing reveals Lgr5+ stem cell activity in mouse intestinal adenomas. Science 337, 730-735, (2012).
3. de Sousa e Melo, F., Kurtova, A. V., Harnoss, J. M., Kljavin, N., Hoeck, J. D., Hung, J. et al. A distinct role for Lgr5(+) stem cells in primary and metastatic colon cancer. Nature 543, 676-680, (2017).
4. Tian, H., Biehs, B., Warming, S., Leong, K. G., Rangell, L., Klein, O. D. et al. A reserve stem cell population in small intestine renders Lgr5-positive cells dispensable. Nature 478, 255-259, (2011).
5. Ben-Sahra, I., Hoxhaj, G., Ricoult, S. J. H., Asara, J. M. & Manning, B. D. mTORC1 induces purine synthesis through control of the mitochondrial tetrahydrofolate cycle. Science 351, 728-733, (2016).
6. Stine, Z. E., Walton, Z. E., Altman, B. J., Hsieh, A. L. & Dang, C. V. MYC, Metabolism, and Cancer. Cancer Discov 5, 1024-1039, (2015).
7. Huang, F., Ni, M., Chalishazar, M. D., Huffman, K. E., Kim, J., Cai, L. et al. Inosine Monophosphate Dehydrogenase Dependence in a Subset of Small Cell Lung Cancers. Cell Metab 28, 369-382 e365, (2018).
8. Valvezan, A. J., Turner, M., Belaid, A., Lam, H. C., Miller, S. K., McNamara, M. C. et al. mTORC1 Couples Nucleotide Synthesis to Nucleotide Demand Resulting in a Targetable Metabolic Vulnerability. Cancer Cell 32, 624-638 e625, (2017).
9. Chen, J., Chaurio, R. A., Maueroder, C., Derer, A., Rauh, M., Kost, A. et al. Inosine Released from Dying or Dead Cells Stimulates Cell Proliferation via Adenosine Receptors. Front Immunol 8, 504, (2017).
10. Gregory, C. D. & Pound, J. D. Cell death in the neighbourhood: direct microenvironmental effects of apoptosis in normal and neoplastic tissues. J Pathol 223, 177-194, (2011).
11. Rock, K. L. & Kono, H. The inflammatory response to cell death. Annu Rev Pathol 3, 99-126, (2008).
12. Greten, F. R. & Grivennikov, S. I. Inflammation and Cancer: Triggers, Mechanisms, and Consequences. Immunity 51, 27-41, (2019).
13. Chen, Q., Sun, L. & Chen, Z. J. Regulation and function of the cGAS-STING pathway of cytosolic DNA sensing. Nat Immunol 17, 1142-1149, (2016).
14. Ahn, J., Xia, T., Konno, H., Konno, K., Ruiz, P. & Barber, G. N. Inflammation-driven carcinogenesis is mediated through STING. Nat Commun 5, 5166, (2014).
15. Bakhoum, S. F., Ngo, B., Laughney, A. M., Cavallo, J. A., Murphy, C. J., Ly, P. et al. Chromosomal instability drives metastasis through a cytosolic DNA response. Nature 553, 467-472, (2018).
16. Chen, Q., Boire, A., Jin, X., Valiente, M., Er, E. E., Lopez-Soto, A. et al. Carcinomaastrocyte gap junctions promote brain metastasis by cGAMP transfer. Nature 533, 493-498, (2016).
17. Lemos, H., Mohamed, E., Huang, L., Ou, R., Pacholczyk, G., Arbab, A. S. et al. STING Promotes the Growth of Tumors Characterized by Low Antigenicity via IDO Activation. Cancer Res 76, 2076-2081, (2016).
18. Haag, S. M., Gulen, M. F., Reymond, L., Gibelin, A., Abrami, L., Decout, A. et al. Targeting STING with covalent small-molecule inhibitors. Nature 559, 269-273, (2018).
19. Di Virgilio, F. & Adinolfi, E. Extracellular purines, purinergic receptors and tumor growth. Oncogene 36, 293-303, (2017).
20. Burnstock, G. Purinergic Signalling: Therapeutic Developments. Front Pharmacol 8, 661, (2017).
21. Shimokawa, M., Ohta, Y., Nishikori, S., Matano, M., Takano, A., Fujii, M. et al. Visualization and targeting of LGR5(+) human colon cancer stem cells. Nature 545, 187-192, (2017).
22. Schwitalla, S., Fingerle, A. A., Cammareri, P., Nebelsiek, T., Goktuna, S. I., Ziegler, P.K. et al. Intestinal tumorigenesis initiated by dedifferentiation and acquisition of stemcell-like properties. Cell 152, 25-38, (2013).
23. Wang, D., Fu, L., Sun, H., Guo, L. & DuBois, R. N. Prostaglandin E2 Promotes Colorectal Cancer Stem Cell Expansion and Metastasis in Mice. Gastroenterology 149, 1884-1895 e1884, (2015).
24. Gehart, H. & Clevers, H. Tales from the crypt: new insights into intestinal stem cells. Nat Rev Gastroenterol Hepatol 16, 19-34, (2019).
25. Degirmenci, B., Valenta, T., Dimitrieva, S., Hausmann, G. & Basler, K. GLI1-expressing mesenchymal cells form the essential Wnt-secreting niche for colon stem cells. Nature 558, 449-453, (2018).
26. Harnack, C., Berger, H., Antanaviciute, A., Vidal, R., Sauer, S., Simmons, A. et al. Rspondin 3 promotes stem cell recovery and epithelial regeneration in the colon. Nat Commun 10, 4368, (2019).
27. Canli, O., Nicolas, A. M., Gupta, J., Finkelmeier, F., Goncharova, O., Pesic, M. et al. Myeloid Cell-Derived Reactive Oxygen Species Induce Epithelial Mutagenesis. Cancer Cell 32, 869-883 e865, (2017).
28. Greten, F. R., Eckmann, L., Greten, T. F., Park, J. M., Li, Z. W., Egan, L. J. et al. IKKbeta links inflammation and tumorigenesis in a mouse model of colitis-associated cancer. Cell 118, 285-296, (2004).
29. Chaffer, C. L., Marjanovic, N. D., Lee, T., Bell, G., Kleer, C. G., Reinhardt, F. et al. Poised chromatin at the ZEB1 promoter enables breast cancer cell plasticity and enhances tumorigenicity. Cell 154, 61-74, (2013).
30. Gupta, P. B., Fillmore, C. M., Jiang, G., Shapira, S. D., Tao, K., Kuperwasser, C. et al. Stochastic state transitions give rise to phenotypic equilibrium in populations of cancer cells. Cell 146, 633-644, (2011).
31. Vermeulen, L., De Sousa, E. M. F., van der Heijden, M., Cameron, K., de Jong, J. H., Borovski, T. et al. Wnt activity defines colon cancer stem cells and is regulated by the microenvironment. Nat Cell Biol 12, 468-476, (2010).
32. Faller, W. J., Jackson, T. J., Knight, J. R., Ridgway, R. A., Jamieson, T., Karim, S. A. et al. mTORC1-mediated translational elongation limits intestinal tumour initiation and growth. Nature 517, 497-500, (2015).
33. Gupta, J., del Barco Barrantes, I., Igea, A., Sakellariou, S., Pateras, I. S., Gorgoulis, V. G. et al. Dual function of p38alpha MAPK in colon cancer: suppression of colitis-associated tumor initiation but requirement for cancer cell survival. Cancer Cell 25, 484-500, (2014).
34. Greten, F. R., Arkan, M. C., Bollrath, J., Hsu, L. C., Goode, J., Miething, C. et al. NFkappaB is a negative regulator of IL-1beta secretion as revealed by genetic and pharmacological inhibition of IKKbeta. Cell 130, 918-931, (2007).
35. Pallangyo, C. K., Ziegler, P. K. & Greten, F. R. IKKbeta acts as a tumor suppressor in cancer-associated fibroblasts during intestinal tumorigenesis. J Exp Med 212, 2253-2266, (2015).
36. Fellmann, C., Hoffmann, T., Sridhar, V., Hopfgartner, B., Muhar, M., Roth, M. et al. An optimized microRNA backbone for effective single-copy RNAi. Cell Rep 5, 1704-1713, (2013).
37. Mali, P., Yang, L., Esvelt, K. M., Aach, J., Guell, M., DiCarlo, J. E. et al. RNA-guided human genome engineering via Cas9. Science 339, 823-826, (2013).
38. Drost, J., van Jaarsveld, R. H., Ponsioen, B., Zimberlin, C., van Boxtel, R., Buijs, A. et al. Sequential cancer mutations in cultured human intestinal stem cells. Nature 521, 43-47, (2015).
39. Susaki, E. A., Tainaka, K., Perrin, D., Kishino, F., Tawara, T., Watanabe, T. M. et al. Whole-brain imaging with single-cell resolution using chemical cocktails and computational analysis. Cell 157, 726-739, (2014).
40. Hoette, K., Koch, M., Hof, L., Tuppi, M., Moreth, T., Stelzer, E. H. K. et al. Ultrathin fluorocarbon foils optimize multiscale imaging of three-dimensional native and optically cleared specimens. bioRxiv, 533844, (2019).
41. Schindelin, J., Arganda-Carreras, I., Frise, E., Kaynig, V., Longair, M., Pietzsch, T. et al. Fiji: an open-source platform for biological-image analysis. Nat Methods 9, 676-682, (2012).
42. Fehrenbach, J., Weiss, P. & Lorenzo, C. Variational algorithms to remove stationary noise: applications to microscopy imaging. IEEE Trans Image Process 21, 4420-4430, (2012).

## Claims

1. An effective amount of an inhibitor of the P2X4 receptor or an inhibitor of the P2X4 receptor signaling pathway in combination with an effective amount of a cell death inducing chemotherapeutic drug and/or cell death inducing therapy for use in the prevention and/or treatment of a solid tumor or metastases thereof in a subject.

2. The inhibitor of the P2X4 receptor or inhibitor of the P2X4 receptor signaling pathway in combination with the cell death inducing chemotherapeutic drug and/or cell death inducing therapy for use according to claim 1, wherein said inhibitor of the P2X4 receptor is a specific inhibitor of the P2X4 receptor, and is preferably selected from the group of PPADS, Suramin, KN-62, TNP-ATP, Brilliant Blue G, 5-BDBD, BX-430, Carbamazepine der., PSB-12054, PSB-12062, PSB-15417, NP-1815-PX, NC-2600, UoS14919, Paroxetine, Duloxetine, BAY-1797, IgG#151-LO, an antibody or a fragment or derivative thereof, a siRNA, a shRNA, a miRNA, a ribozyme, an aptamer, an antisense nucleic acid molecule, a small molecule or a modified version of these inhibitors, preferably 5DBDB.

3. The inhibitor of the P2X4 receptor or inhibitor of the P2X4 receptor signaling pathway in combination with the cell death inducing chemotherapeutic drug and/or cell death inducing therapy for use according to claim 1 or 2, wherein said drug is selected from the group of necrotic cell supernatants; cationic amphiphilic drugs (CAD); classical anticancer agents, including anthracyclines, antimetabolites, and platinum drugs; topoisomerase inhibitors, e.g. camptothecin; PDE3A inhibitors, such as anagrelide, either alone or with cell death-inducing cytokines; death-inducing cytokines, such as IFN-α, IFN-γ, TNF-α, or TRAIL; taxanes, paclitaxel, and fluorinated taxanes, such as SB-T-12851, SB-T-12852, SB-T-12853, SB-T-12854, 5-fluorouracil, tegafur, capecitabine, and doxifluridine, and preferably 5-fluorouracil (5-FU).

4. The inhibitor of the P2X4 receptor or inhibitor of the P2X4 receptor signaling pathway in combination with the cell death inducing chemotherapeutic drug and/or cell death inducing therapy for use according to any one of claims 1 to 3, wherein said a solid tumor or metastases thereof is selected from Lrp5+ cancer stem cell-associated cancers, prostate cancer, pancreatic cancer, breast cancer, gastric cancer, liver cancer, brain cancer, lung cancer, kidney cancer, and colorectal cancer, and metastases thereof, in particular colorectal cancer, and metastases thereof.

5. The inhibitor of the P2X4 receptor or inhibitor of the P2X4 receptor signaling pathway in combination with the cell death inducing chemotherapeutic drug and/or cell death inducing therapy for use according to any one of claims 1 to 4, wherein said treatment and/or prevention further comprises a prior and/or concomitant anti-cancer chemotherapy, comprising for example cellular toxins, Lgr5 inhibitors, Lgr5+ cancer cell ablation, mTOR inhibitors, and IMPDH inhibitors.

6. The inhibitor of the P2X4 receptor or inhibitor of the P2X4 receptor signaling pathway in combination with the cell death inducing chemotherapeutic drug and/or cell death inducing therapy for use according to any one of claims 1 to 5, wherein said combination is provided simultaneously or separately, as combined and/or separate dosage forms.

7. The inhibitor of the P2X4 receptor or inhibitor of the P2X4 receptor signaling pathway in combination with the cell death inducing chemotherapeutic drug and/or cell death inducing therapy for use according to any one of claims 1 to 6, wherein said treatment and/or prevention comprises the inhibition of the ATP-dependent P2X4 receptor- and/or P2X4 receptor signaling pathway-mediated tumor escape mechanism.

8. A method of preventing and/or treating a solid tumor or metastases thereof in a subject in need thereof, the method comprising the concomitant or sequential administration of (i) an effective amount of an inhibitor of the P2X4 receptor or an inhibitor of the P2X4 receptor signaling pathway, and (ii) an effective amount of a cell death inducing chemotherapeutic drug and/or a cell death inducing therapy to said subject.

9. The method according to claim 8, wherein said inhibitor of the P2X4 receptor is a specific inhibitor of the P2X4 receptor, and is preferably selected from the group of PPADS, Suramin, KN-62, TNP-ATP, Brilliant Blue G, 5-BDBD, BX-430, Carbamazepine der., PSB-12054, PSB-12062, PSB-15417, NP-1815-PX, NC-2600, UoS14919, Paroxetine, Duloxetine, BAY-1797, IgG#151-LO, an antibody or a fragment or derivative thereof, a siRNA, a shRNA, a miRNA, a ribozyme, an aptamer, an antisense nucleic acid molecule, a small molecule or a modified version of these inhibitors, preferably 5DBDB.

10. The method according to claim 8 or 9, wherein said drug is selected from the group of necrotic cell supernatants; cationic amphiphilic drugs (CAD); classical anticancer agents, including anthracyclines, antimetabolites, and platinum drugs; topoisomerase inhibitors, e.g. camptothecin; PDE3A inhibitors, such as anagrelide, either alone or with cell death-inducing cytokines; cancer cell apoptosis inducing compounds; death-inducing cytokines, such as IFN-α, IFN-γ, TNF-α, or TRAIL; taxanes, paclitaxel, and fluorinated taxanes, such as SB-T-12851, SB-T-12852, SB-T-12853, SB-T-12854, 5-fluorouracil, tegafur, capecitabine, and doxifluridine, and preferably 5-fluorouracil (5-FU).

11. The method according to any one of claims 8 to 10, wherein said treatment and/or prevention comprises the inhibition of the ATP-dependent P2X4 receptor- and/or P2X4 receptor signaling pathway-mediated tumor escape mechanism(s).

12. The method according to any one of claims 8 to 11, wherein said treatment and/or prevention further comprises a prior and/or concomitant anti-cancer chemotherapy, comprising for example cellular toxins, Lgr5 inhibitors, Lgr5+ cancer cell ablation, mTOR inhibitors, and IMPDH inhibitors.
